(19) Europäisches Patentamt
European Patent Office
Office européen des brevets

(11) EP 4 499 024 B1

(12) # FASCICULE DE BREVET EUROPEEN

(45) Date de publication et mention de la délivrance du brevet:
**18.02.2026 Bulletin 2026/08**

(21) Numéro de dépôt: **23713116.4**

(22) Date de dépôt: **23.03.2023**

(51) Classification Internationale des Brevets (IPC):
***A61K 8/06*** *(2006.01)* ***A61K 8/31*** *(2006.01)*
***A61K 8/81*** *(2006.01)* ***A61Q 5/06*** *(2006.01)*

(52) Classification Coopérative des Brevets (CPC):
**A61Q 5/06; A61K 8/062; A61K 8/064; A61K 8/31; A61K 8/8111**

(86) Numéro de dépôt international:
**PCT/EP2023/057436**

(87) Numéro de publication internationale:
**WO 2023/180433 (28.09.2023 Gazette 2023/39)**

(54) **UTILISATION EN COSMETIQUE DE POLYFARNESENES PARTIELLEMENT OU TOTALEMENT HYDROGENES ET PROCEDE DE TRAITEMENT COSMETIQUE DES CHEVEUX**

KOSMETISCHE VERWENDUNG VON TEIL- ODER VOLLHYDRIERTEN POLYFARNESEN UND VERFAHREN ZUR KOSMETISCHEN BEHANDLUNG VON HAAREN

COSMETIC USE OF PARTIALLY OR TOTALLY HYDROGENATED POLYFARNESENES, AND METHOD FOR THE COSMETIC TREATMENT OF HAIR

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **24.03.2022 FR 2202634**

(43) Date de publication de la demande:
**05.02.2025 Bulletin 2025/06**

(73) Titulaires:
- **Société d’Exploitation de Produits pour les Industries Chimiques SEPPIC 75321 Paris cedex 07 (FR)**
- **TotalEnergies OneTech 92400 Courbevoie (FR)**

(72) Inventeurs:
- **CUQ ARNAUD, Elodie 81100 CASTRES (FR)**
- **FRYSCHER, Jennifer 92250 LA GARENNE COLOMBES (FR)**

(74) Mandataire: **Alatis 3, rue Paul Escudier 75009 Paris (FR)**

(56) Documents cités:
**WO-A1-2020/144440 DE-A1- 10 036 329**
**US-A1- 2019 142 736**

- **DATABASE GNPD [online] MINTEL; 1 August 2018 (2018-08-01), ANONYMOUS: "Thermoprotective Smoothing Spray", XP055982416, retrieved from https://www.gnpd.com/sinatra/recordpage/5873453/ Database accession no. 5873453**

Il est rappelé que: Dans un délai de neuf mois à compter de la publication de la mention de la délivrance du brevet européen au Bulletin européen des brevets, toute personne peut faire opposition à ce brevet auprès de l’Office européen des brevets, conformément au règlement d’exécution. L’opposition n’est réputée formée qu’après le paiement de la taxe d’opposition. (Art. 99(1) Convention sur le brevet européen).

EP 4 499 024 B1

## Description

### Domaine technique de l'invention

La présente invention relève du domaine de la cosmétique. Elle concerne plus particulièrement le traitement cosmétique des cheveux.

### Arrière-plan technologique

Lors du séchage ou du coiffage, les cheveux peuvent être exposés à la chaleur de l'air chaud des sèche-cheveux ou par celle des fers à friser ou à lisser ou encore celle des peignes ou brosses chauffants. Cette exposition induit un stress thermique qui peut causer des modifications des chaînes polypeptidiques de la kératine des cheveux, en affectant plus particulièrement leurs conformations stéréochimiques et certaines liaisons intermoléculaires, et par conséquent en altérant leur structure interne. Une telle altération entraîne à la fois une dégradation de leur apparence, par exemple une diminution de leur brillance, et une dégradation de leurs propriétés sensorielles, par exemple une moindre douceur. De plus il est parfois constaté des dommages externes des cheveux, comme le soulèvement des cuticules et/ou la création de cloques sur les fibres capillaires individuelles ce qui le fragilise en détériorant sa surface, voire en provoquant sa rupture, à cause de l'augmentation de la friction entre les fibres capillaires qui en résulte. De nombreuses compositions ont été développées et commercialisées pour protéger les cheveux des effets négatifs associés à l'exposition des cheveux à de fortes températures. Elles forment un revêtement sur les fibres capillaires et diminuent ainsi l'effet de la chaleur en leur sein. Ces compositions comprennent des polymères filmogènes, comme ceux issus de la polymérisation de la N-vinyl pyrrolidones, d'alcools vinyliques ou de l'acétate de vinyle.

La demande internationale publiée sous le numéro WO 2014/95210A1 divulgue une composition comprenant au moins un polymère non ionique, comme un mélange de copolymères issus de la N-vinyl pyrrolidones et/ou de l'acétate de vinyle, avec au moins un polymère cationique par exemple Polyquaternium-46. La présence du polymère cationique sert à diminuer ou supprimer la sensation collante induite par le polymère filmogène et à limiter la présence de résidus blancs inesthétiques sur les cheveux.

Des dérivés de silicone sont également connus comme ingrédients multifonctionnels dans les compositions de soins capillaires pour, entre autres propriétés, prévenir contre les dommages générés par la chaleur. Il y a par exemple les copolymères de type « aminoglycols » comme le bis(C13-C15 alkoxy) PG-amodimethicone, commercialisé sous le nom : « Dow Corning 8500 conditionning agent. Les compositions de soins capillaires en comportant, sont stables, s'étalent aisément sur les cheveux et forment un film protecteur qui empêche la perte d'eau due à l'exposition à la chaleur, au niveau de la tige des cheveux.

Les polymères de la famille des siloxanes sont aussi connus pour protéger les cheveux exposés à la chaleur. Parmi ceux-ci, il y a par exemple le poly(diméthylsiloxane) de formule : $-[O-Si(CH_3)_2]_n-$, communément appelé PDMS ou diméthicone, ou les diméthiconols se caractérisant par une structure de formule : $HO-Si(CH_3)_2-[O-Si(CH_3)_2]_n-Si(CH_3)_2-OH$. Les mélanges de diméthicone et de diméthiconol sont très utilisés dans des formules d'hygiène (notamment les shampoings) et de soins capillaires.

Comme composés siliconés assurant une protection des cheveux exposés à la chaleur, il y a encore ceux de la famille des aminosilanes comme ceux décrits dans les demandes de brevet français publiée sous le numéro FR 3,038,513 A1 , et américain publiée sous le numéro US 2018/161266 A2. La demande internationale publiée sous le numéro WO 2021/260486 A1, divulgue par exemple des compositions comprenant un polymère filmogène choisi parmi le Polysilicone-35, le Polysilicone-33, le Polysilicone-29, le Polysilicone-28, le Polysilicone-25, le Polysilicone-24, le Polysilicone-22, le Polysilicone-19, le Polysilicone-18, le Polysilicone-14 et le Polysilicone-3, seuls ou en mélange et un dérivé de silane d'une protéine hydrolysée ou d'un peptide ou d'un acide aminé.

Cependant, les polymères susmentionnés ne sont que peu ou pas biodégradables et sont issus de matières premières d'origine fossile.

Les inventeurs se sont donc attachés à développer une nouvelle solution alternative aux copolymères précédemment cités qui, tout en assurant une protection suffisante des cheveux lors de leur exposition à la chaleur, a des caractéristiques environnementales conformes à la fois aux réglementations en vigueur et à venir et aux exigences des consommateurs.

### Exposé de l'invention

Selon un premier aspect, l'invention a pour objet l'utilisation d'une composition (C) comprenant pour 100% de sa masse,

- de 10% massique à 90% massique d'au moins un polymère poly(farnésène) ayant une masse molaire moyenne en

nombre supérieure ou égale 10.000 g/mol et inférieure ou égale à 120.000 g/mol, ledit polymère poly(farnésène) étant partiellement ou totalement hydrogéné et/ou fonctionnalisé avec des radicaux hydroxyle, et

- de 10% massique à 90% massique d'un mélange (M) d'hydrocarbures dans lequel au moins 94% massique des dits hydrocarbures comportent de quinze à dix-neuf atomes de carbone,

pour empêcher et/ou ralentir l'apparition d'effets inesthétiques sur les cheveux humains suite à l'exposition à une chaleur intense.

Par « effets inesthétiques sur les cheveux humains », on entend au sens de la présente invention, toute modification de l'aspect extérieur due aux conséquences de l'exposition à une chaleur intense comme le soulèvement des cuticules, l'apparition de cloques sur les fibres capillaires individuelle, le ternissement, la fragilisation des cheveux vis-à-vis de contraintes mécaniques, l'apparition de fourches, l'apparition de fissures et de cassures dans les cheveux, la perte de douceur, l'augmentation de la porosité de la fibre et/ou la diminution de l'hydrophobicité du cheveu.

Par « chaleur intense », on entend au sens de la présente invention, la chaleur induite émise par les appareils utilisés pour sécher, lisser et/ou friser des cheveux humides, tels qu'un sèche-cheveux, un casque à sécher, un peigne chauffant, une brosse à cheveux chauffante, un fer à lisser ou un fer à friser. Cette dite chaleur intense est émise lorsque ces appareils fonctionnent à une température généralement comprise entre 50°C et 250°C.

Dans le cadre de la présente invention, le ou les polymères poly(farnésène) compris dans la composition (C) telle que définie ci-dessus, sont connus et leur structure chimique est explicitée dans la demande internationale publiée sous le numéro WO 2020/144440 A1. Ils peuvent être obtenus par le procédé décrit dans la demande internationale publiée sous le numéro WO 2018/052709 A1.

Dans le cadre de la présente invention, on désigne par « mélange (M) d'hydrocarbures », un mélange d'alcanes ramifiés, et/ou d'alcanes linéaires et/ou de cycloalcanes. Par « alcanes linéaire » comportant de quinze à dix-neuf atomes de carbone, on désigne le n-pentadécane, le n-hexadécane, le n-heptadécane, le n-octadécane et le n-nonadécane ; par « alcanes ramifiés » (ou iso-alcanes) comportant de quinze à dix-neuf atomes de carbone, on désigne plus particulièrement le 2-méthyl tétradécane (ou isopentadécane), le 2-méthyl pentadécane (ou isohexadécane), le 2-méthyl hexadécane (ou isoheptadécane), le 2-méthyl heptadécane (ou iso-octadécane) et le 2-méthyl octadécane (ou isononadécane) ; par "cyclo-alcanes comportant de quinze à dix-neuf atomes de carbone, on désigne plus particulièrement des hydrocarbures saturés comprenant au moins un groupe hydrocarboné cyclique saturé optionnellement substitué par un ou plusieurs radicaux alkyles linéaires ou ramifiés. Ces mélanges peuvent être obtenus par conversion d'une biomasse comme divulgué dans WO 2020/144440 A1.

Selon un mode particulier de la présente invention, le mélange (M) compris dans la composition (C) telle que définie ci-dessus, comprend pour 100% de sa masse :

- de 15% à 20% massique d'alcanes (linéaires, iso-alcanes et cycloalcanes) comportant 17 atomes de carbone,
- de 70% à 75% massique d'alcanes (linéaires, iso-alcanes et cycloalcanes) comportant 18 atomes de carbone, et
- de 4% à 6% massique d'alcanes (linéaires, iso-alcanes et cycloalcanes) comportant 19 atomes de carbone.

Selon un mode plus particulier de la présente invention, le mélange (M) compris dans la composition (C) telle que définie ci-dessus, est un mélange d'hydrocarbures saturés comprenant, pour 100% de sa masse :

- 3,8% d'alcanes linéaires comportant de quinze à dix-neuf atomes de carbone,
- 96,2% d'iso-alcanes comportant de quinze à dix-neuf atomes de carbone, et
- moins de 0,1% de cycloalcanes comportant de quinze à dix-neuf atomes de carbone,

et dans lequel :

- 15% à 20% massique d'alcanes (linéaires, iso-alcanes et cycloalcanes) comportant 17 atomes de carbone,
- 70% à 75% massique d'alcanes (linéaires, iso-alcanes et cycloalcanes) comportant 18 atomes de carbone, et
- 4% à 6% massique d'alcanes (linéaires, iso-alcanes et cycloalcanes) comportant 19 atomes de carbone.

Dans le cadre de la présente invention le ou les polymères poly(farnésène) compris dans la composition (C) telle que définie ci-dessus, ont plus particulièrement une masse molaire en nombre supérieure ou égale à 20.000 g/mol et inférieure ou égale à 85.000 g/mol, encore plus particulièrement supérieure ou égale à 30.000 g/mol et inférieure ou égale à 80.000 g/mol.

Selon un mode tout particulier de la présente invention, le polymère poly(farnésène) compris dans la composition (C) telle que définie ci-dessus, a une masse molaire moyenne en nombre de 71.000 g/mol et il est totalement hydrogéné et fonctionnalisé avec des radicaux hydroxyle.

L'invention a tout particulièrement pour objet l'utilisation telle que définie ci-dessus, caractérisée en ce que ladite

composition (C) comprend pour 100% de sa masse,

- 40% massique d'un polymère poly(farnésène) totalement hydrogéné, fonctionnalisé avec des groupes hydroxyle et avec des groupes hydroxyle et ayant une masse molaire moyenne en nombre égale à 71.000, et
- 60% massique d'un mélange (M) un mélange d'hydrocarbures saturés comprenant, pour 100% de sa masse :
  - 3,8% d'alcanes linéaires comportant de quinze à dix-neuf atomes de carbone,
  - 96,2% d'iso-alcanes comportant de quinze à dix-neuf atomes de carbone, et
  - moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

La composition (C) telle que définie ci-dessus, peut être préparée par simple mélange dudit polymère poly(farnésène) et du mélange (M), à une température comprise entre 20°C et 60°C, sous agitation mécanique muni d'un mobile de type ancre ou turbine, à une vitesse comprise entre 20 tours/minute et 500 tours/minute.

Selon un autre aspect particulier, l'invention a pour objet l'utilisation telle que définie précédemment, caractérisée en ce que ladite composition (C) est mise en œuvre au sein d'une formulation cosmétique (F) en comprenant de 0,2% à 40% massique pour 100% de sa masse ; et plus particulièrement l'utilisation telle que définie ci-dessus caractérisée en ce que ladite formulation (F) est une émulsion (E) d'une phase aqueuse (A) et d'une phase grasse (G) comprenant pour 100% de sa masse :

- de 50% à 98% massique, de ladite phase aqueuse (A), et
- de 2% à 50% massique, de ladite phase grasse (G),

ladite phase grasse (G) comprenant, pour 100% de sa masse :

- de 10% à 80% massique, de ladite composition (C),
- de 0,5% à 20% massique d'un agent tensioactif émulsionnant (S) ou d'un mélange d'agents tensioactifs émulsionnants ($M_S$) du type huile-dans-eau ou du type eau-dans huile, et
- de 0% à 89,5% massique, d'une huile ou de plusieurs huiles et/ou d'une cire, ladite cire étant différente de ladite composition (C).

Selon cet aspect particulier, l'huile ou les huiles optionnellement comprises dans la phase grasse (G) telle que définie ci-dessus, sont les substances chimiques ou les mélanges de substances chimiques insolubles dans l'eau, et se présentant sous un aspect liquide à une température de 25°C. ces huiles peuvent être d'origine animale, d'origine végétale, ou être des huiles de synthèse.

Comme huile d'origine animale mise en œuvre dans le cadre de la présente invention, il y a par exemple le squalène ou le squalane ;

Comme huile d'origine végétale mise en œuvre dans le cadre de la présente invention, il y a par exemple le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sisymbre, l'huile d'avocat, l'huile de calendula, l'huile de sésame, l'huile de reine des prés, l'huile de Macadamia kiwi, l'huile de bourrache, l'huile de pépins de cassis, l'huile de café, l'huile de pistache, l'huile de noyau de pêche, l'huile de pépin de framboise, l'huile de pépin de fraise, l'huile de melon, l'huile de pépin de myrtille, l'huile d'argan, l'extrait huileux de prune, l'huile de grenade, l'huile de papaye, l'huile de lait de coco, les huiles issues de fleurs ou de légumes et les huiles végétales éthoxylées ; Comme huile synthétique mise en œuvre dans le cadre de la présente invention, il y a par exemple les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle ; les esters dérivés d'acide lanolique, tels que le lanolate d'isopropyle, le lanolate d'isocétyle, le triheptanoate de glycérol, les alkylbenzoates, les huiles hydrogénées, les polyalphaoléfines, les polyoléfines comme le polyisobutène, le polydécène hydrogéné ou le polyisobutène hydrogéné, commercialisé en France par la société Ets B. Rossow et Cie sous le nom PARLEAM-POLYSYNLANE™, cité dans : Michel and Irene Ash ; Thesaurus of Chemical Products, Chemical Publishing Co, Inc. 1986 Volume I, page 211 (ISBN 0 7131 3603 0) ; les huiles de silicone comme les diméthylpolysiloxanes, les méthylphénylpolysiloxanes, les silicones modifiées par des amines, les silicones modifiées par des acides gras, les silicones modifiées par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Selon cet aspect particulier, la cire optionnellement comprise dans la phase grasse (G) telle que définie ci-dessus, est une substance chimique ou un mélange de substances chimiques insolubles dans l'eau et se présentant sous un aspect solide à une température de 45°C. Il y a par exemple la cire d'abeille, la cire de carnauba, la cire de candelilla, la cire d'ourichoury, la cire du Japon, la cire de fibre de liège, la cire de canne à sucre, la cire de jojoba, la cire de fleurs de cassis, la cire de fleurs de narcisse, la cire de fleurs d'oranger, la cire d'orange, la cire de riz, les cires de lignite, les cires microcristallines, la cire de lanoline ; l'ozokérite ; la cire de polyéthylène, les cires de silicone ; les cires végétales ; les alcools gras et les acides gras solides à température ambiante et les glycérides solides à température ambiante.

Selon cet aspect particulier, et selon la nature de l'agent tensioactif émulsionnant (S) ou du mélange d'agents tensioactifs émulsionnants ($M_S$), l'émulsion (E) telle que définie ci-dessus est soit une émulsion de type huile-dans eau (H/E) soit de type eau-dans-huile (E/H).

Par agent tensioactif émulsionnant ($S_1$) du type huile-dans-eau ou mélange d'agents tensioactifs émulsionnants ($M_{S1}$) du type huile-dans-eau présent dans la phase grasse ($G_1$) de l'émulsion ($E_1$) de type (H/E), on désigne une substance chimique ou un mélange de substances chimiques qui permet de stabiliser les gouttelettes de ladite phase grasse ($G_1$) en dispersion dans la phase aqueuse continue (A). Comme exemples dudit tensioactif émulsionnant ($S_1$) ou dudit mélange d'agents tensioactifs émulsionnants ($M_{S1}$), il y a notamment :

- Les polysorbates résultant de la réaction d'éthoxylation entre un équivalent molaire d'esters de sorbitan, tels que le laurate de sorbitan, le palmitate de sorbitan, le stéarate de sorbitan, l'isostéarate de sorbitan ou l'oléate de sorbitan avec 5 à 20 équivalents molaires d'oxyde d'éthylène ;
- Les produits résultant de la réaction d'éthoxylation entre un équivalent molaire d'un acide gras, tel que l'acide palmitique, l'acide myristique, l'acide laurique, l'acide stéarique, l'acide isostéarique ou l'acide oléique, avec 5 à 40 équivalents molaires d'oxyde d'éthylène ;
- Les produits résultant de la réaction d'estérification entre un acide gras, tel que l'acide palmitique, l'acide myristique, l'acide laurique, l'acide stéarique, l'acide isostéarique, l'acide oléique, l'acide arachidique ou l'acide béhénique avec 3 à 20 équivalents molaires de glycérol ;

Parmi les tensioactifs émulsionnants de type eau-dans-huile ($S_2$) susceptibles d'être utilisés dans le cadre de la présente invention, il y a notamment :

- les lipoaminoacides et leurs sels et les lipopeptides et leurs sels ; les esters de sorbitan comme les laurate, palmitate, stéarate, oléate, sesquioléate, trioléate, et isostéarate de sorbitan ; les esters de glycérol comme les laurate, palmitate, stéarate, oléate, sesquioléate, trioléate et isostéarate de glycérol ;
- les esters de polyglycol, de polyglycérol ou de polyols comme les laurate, palmitate, stéarate, oléate, sesquioléate, trioléate, l'isostéarate, les polyhydroxystéarates de polyglycols ou de polyglycérol tels que les HYPERMER™ B246, ARLACEL™ P135, DEHYMULS™ PGPH et DECAGLYN™ 5HS,
- les copolymères polyéthylèneglycol-alkylglycol comme le PEG-45 dodécylglycol copolymère tel que l'ELFACOS ST 9™ ;
- les esters de sucrose, les esters de méthylglucoside ethoxylés ou non,
- les acides gras et les alcools gras éthoxylés ;
- les émulsionnants anioniques comme le décylphosphate ou le cétéarylsulfate ; le polyoxystéarate d'aluminium, comme le MANALOX®, le stéarate de magnésium et le stéarate d'aluminium ; et
- les alkylpolyglycosides, les compositions d'alkylpolyglycosides et d'alcools gras.

Comme exemple de compositions d'alkylpolyglycosides, il y a la composition ($CA_1$) représentée par la formule : $R_1$-O-$(G)_x$-H dans laquelle G représente le reste d'un sucre réducteur choisi parmi le glucose ou le xylose, $R_1$-O- est lié à G par le carbone anomérique du reste saccharide, de manière à former une fonction acétal, x représente un nombre décimal supérieur ou égal à 1,05 et inférieur ou égal à 2,5 et $R_1$ représente un radical aliphatique choisi parmi les radicaux les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle et 2-décyl tétradécyle ladite composition ($CA_1$) consistant en un mélange de composés représentés par les formules $R_1$-O-$(G)_1$-H, $R_1$-O-$(G)_2$-H, $R_1$-O-$(G)_3$-H et $R_1$-O-$(G)_4$-H et $R_1$-O-$(G)_5$-H, dans les proportions molaires respectives $a_1$, $a_2$, $a_3$, $a_4$ et $a_5$, telles que la somme $a_1+a_2+a_3+a_4+a_5$ est égale à 1 et que la somme $a_1+2a_2+3a_3+4a_4+5a_5$ est égale à x.

Comme exemple de compositions d'alkylpolyglycosides et d'alcools gras, il y a la composition ($CA_2$) comprenant pour 100% de sa masse :

- De 10% à 50% massique et plus particulièrement de 20% à 30% massique de ladite composition ($CA_1$) telle que définie ci-dessus et
- De 90% à 50% massique et plus particulièrement de 80% à 70% massique d'au moins un alcool gras de formule : $R'_1$-OH, dans laquelle $R'_1$, identique ou différent de $R_1$, représente un radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle ou 2-décyl tétradécyle ; $R'_1$ représente plus particulièrement le radical 2-octyl dodécyle.

Comme autre exemple de compositions d'alkylpolyglycosides et d'alcools gras, il y a la composition ($CA_3$) comprenant pour 100% de sa masse :

- de 15 % à 25 % massique de la composition ($CA_1$),
- de 55 % à 65 % massique d'au moins un alcool gras de formule R'1-OH dans laquelle R'1 représente le radical 2-octyl dodécyle ; et

de 10 % à 30 % massique d'au moins un poly(hydroxystéarate) de polyglycol représenté par la formule $Z_2$-O-[$CH_2$-CH($R_4$)-O]$_{y2}$-$Z'_2$, dans laquelle y2 représente un nombre entier supérieur ou égal à 2 et inférieur ou égal à 50, $R_4$ représente l'atome d'hydrogène, le radical méthyle ou le radical éthyle, $Z_2$ représente un radical de formule :

[Chem 1]

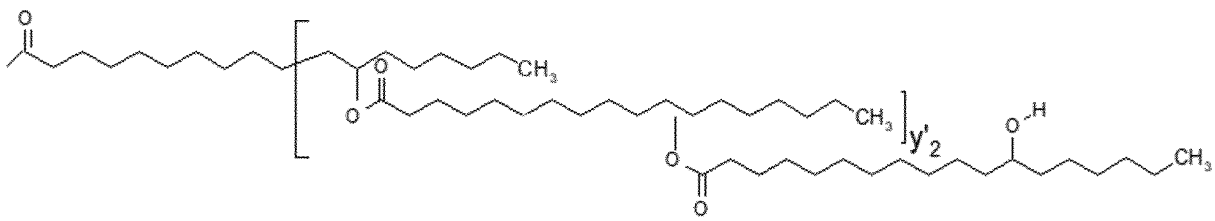

dans laquelle $y'_2$ représente un nombre entier supérieur ou égal à 0 et inférieur ou égal à 10, plus particulièrement supérieur ou égal à 1 et inférieur ou égal à 10 et $Z'_2$, identique ou différent de $Z_2$, représente l'atome d'hydrogène ou le radical de formule telle que définie ci-dessus.

Par agent tensioactif émulsionnant ($S_2$) du type (E/H) ou mélange d'agents tensioactifs émulsionnants ($M_{S2}$) du type (E/H) présent dans la phase grasse ($G_2$) d'une émulsion ($E_2$) de type (E/H), il y a plus particulièrement les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés, les copolymères polyéthylèneglycol-alkylglycols.

Ladite émulsion ($E_2$) de type (E/H) telle que définie précédemment, peut également comprendre au sein de ladite phase aqueuse (A) pour 100% de sa masse, de 0,5% massique à 10% massique et de préférence de 0,5% massique à 5% massique d'un polyélectrolyte anionique réticulé (P) ou d'un mélange de polyélectrolytes anioniques réticulés ($M_P$).

Par polyélectrolyte anionique réticulé (P), on désigne, dans la définition précédente, un polyélectrolyte anionique réticulé non linéaire, se présentant à l'état de réseau tridimensionnel insoluble dans l'eau, mais gonflable à l'eau et conduisant à l'obtention d'un gel chimique.

Comme exemples dudit polyélectrolyte anionique réticulé (P), il y a :

- un homopolymère de l'acide 2-méthyl 2-[(1-oxo 2-propènyl) amino] 1-propanesulfonique partiellement ou totalement salifié sous forme de sel de sodium ou de sel d'ammonium, réticulé par le triallylamine et/ou le méthylène-bis(acrylamide) ;
- un copolymère de l'acide 2-méthyl 2-[(1-oxo 2-propènyl) amino] 1-propanesulfonique ($\gamma$), partiellement ou totalement salifié sous forme de sel de sodium ou de sel d'ammonium et de l'acide acrylique ($\delta$) partiellement ou totalement salifié sous forme de sel de sodium ou de sel d'ammonium, réticulé par le triallylamine et/ou le méthylène-bis(acrylamide) ; notamment ledit copolymère dans lequel les proportions molaires en monomères ($\gamma$), et ($\delta$) sont les suivantes :

  $30\% \leq (\gamma) \leq 90\%$ ou plus particulièrement $40\% \leq (\gamma) \leq 90\%$ et

10% ≤ (δ) ≤ 70% ou plus particulièrement 10% ≤ (δ) ≤ 60% ;

- un copolymère de l'acide 2-méthyl 2-[(1-oxo 2-propènyl) amino] 1-propanesulfonique (γ) partiellement ou totalement salifié sous forme de sel de sodium ou de sel d'ammonium et de l'acrylamide (ε), réticulé par le triallylamine et/ou le méthylène-bis(acrylamide) ; notamment ledit copolymère dans lequel les proportions molaires en monomères (γ), et (ζ) sont les suivantes :

$$30\% \leq (\gamma) \leq 90\%,$$

et

$$10\% \leq (\varepsilon) \leq 70\% \text{ ;}$$

- un copolymère de l'acide 2-méthyl 2-[(1-oxo 2-propènyl) amino] 1-propanesulfonique (γ) partiellement ou totalement salifié sous forme de sel de sodium, et de l'hydroxyéthylacrylate (ζ), réticulé par le triallylamine et/ou le méthylène-bis(acrylamide) ; notamment ledit copolymère dans lequel les proportions molaires en monomères (γ), et (ζ) sont les suivantes :

$$30\% \leq (\gamma) \leq 90\%,$$

et

$$10\% \leq (\zeta) \leq 70\% \text{ ;}$$

- un terpolymère de l'acide 2-méthyl 2-[(1-oxo 2-propènyl) amino] 1-propanesulfonique (γ) partiellement ou totalement salifié sous forme de sel de sodium ou de sel d'ammonium, l'acide acrylique (δ) partiellement ou totalement salifiés sous forme de sel de sodium ou de sel d'ammonium et de l'acrylamide (ε), réticulé par le triallylamine et/ou le méthylènebis(acrylamide) ; notamment ledit terpolymère dans lequel les proportions molaires en monomères (γ), (δ) et (ε) sont les suivantes :

$$30\% \leq (\gamma) \leq 45\%,$$

$$2\% \leq (\delta) \leq 10\%,$$

et plus particulièrement 2% ≤ (δ) ≤ 8%, et

$$45\% \leq (\varepsilon) \leq 68\%,$$

et plus particulièrement 47% ≤ (δ) ≤ 68% ;

- un terpolymère de l'acide 2-méthyl 2-[(1-oxo 2- propènyl) amino] 1-propanesulfonique (γ) partiellement ou totalement salifié sous forme de sel de sodium ou de sel d'ammonium, du N,N diméthylacrylamide (β) et du méthacrylate de lauryle tétraéthoxylé (α), réticulé par le triméthylolpropanetriacrylate et/ou le triallylamine et/ou le méthylène-bis(acrylamide) ; notamment ledit terpolymère dans lequel les proportions molaires en monomères (γ), (β) et (α) sont les suivantes :

$$60\% \leq (\gamma) \leq 80\%,$$

$$15\% \leq (\beta) \leq 39{,}5\%,$$

et

$$0{,}5\% \leq (\alpha) \leq 5\% \text{ ;}$$

- un tétrapolymère de l'acide 2-méthyl 2-[(1-oxo 2- propènyl) amino] 1-propanesulfonique (γ) partiellement ou totalement salifié sous forme de sel de sodium ou de sel d'ammonium, du N,N diméthylacrylamide (β), du méthacrylate de lauroyle (η) et du méthacrylate de stéaroyle (μ), réticulé par le trimethylolpropanetriacrylate et/ou le triallylamine et/ou le méthylène-bis(acrylamide) ; notamment ledit tétrapolymère dans lequel les proportions molaires en monomères (γ), (β), (η) et (μ) sont les suivantes :

$$60\% \leq (\gamma) \leq 80\%,$$

$$15\% \leq (\beta) \leq 39\%,$$

$$0{,}5\% \leq (\eta) \leq 2{,}5\%,$$

et

$$0{,}5\% \leq (\mu) \leq 2{,}5\%.$$

Les émulsions (E), (E1) et ($E_2$) peuvent en outre comporter des excipients et/ou des principes actifs habituellement mis en œuvre dans le domaine des formulations destinées à être appliquées sur le cheveu ou le cuir chevelu. C'est pourquoi, selon un autre mode particulier de la présente invention, les émulsions (E), ($E_1$) et ($E_2$), telles que définies précédemment, comprennent en outre un ou plusieurs composés auxiliaires choisis parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques ou les répulsifs pour les insectes.

Comme exemples de tensioactifs moussants et/ou détergents, optionnellement présents dans les émulsions (E), ($E_1$) et ($E_2$) telle que définies ci-dessus, il y a des tensioactifs moussants et/ou détergents anioniques, cationiques, amphotères ou non ioniques topiquement acceptables habituellement utilisés dans ce domaine de la cosmétique.

Parmi les tensioactifs anioniques moussants et/ou détergents « cosmétiquement acceptables », il y a les sels de métaux alcalins, alcalino-terreux, ou ammonium, d'amines, d'amino alcools, d'alkyléthers sulfates, alkyl sulfates, alkylamidoéther sulfates, alkylarylpolyéther sulfates, monoglycéride sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfoacétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, ou d'acides gras.

Parmi les tensioactifs amphotères moussants et/ou détergents « cosmétiquement acceptables », il y a les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents « cosmétiquement acceptables », il y a les dérivés d'ammoniums quaternaires.

Parmi les tensioactifs non ioniques moussants et/ou détergents « cosmétiquement acceptables », il y a les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah et les N-alkyl amines.

Parmi les tensioactifs épaississants et/ou gélifiants « cosmétiquement acceptables », il y a notamment :

- les esters gras d'alkylpolyglycosides optionnellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ;
- les esters gras alcoxylés tels que le PEG 150 pentaérythrytyl tétrastéarate et le PEG 55 propylene glycol oléate commercialisés respectivement sous les appellations CROTHIX™ DS53, et ANTIL™ 141 ;
- les carbamates de polyalkylène glycols à chaînes grasses tels que le PPG 14 laureth isophoryl dicarbamate et PPG 14 palmeth 60 hexyl dicarbamate respectivement commercialisés sous les appellations ELFACOS™ T211 et

ELFACOS™ GT2125.

Parmi les tensioactifs émulsionnants « cosmétiquement acceptables », il y a notamment des tensioactifs non ioniques, des tensioactifs anioniques, des tensioactifs cationiques.

Comme tensioactifs non ioniques émulsionnants « cosmétiquement acceptables », on peut citer les esters d'acides gras et de sorbitol, par exemple les produits commercialisés sous les appellations MONTANE™80 et MONTANE™85 et MONTANE™60 ; l'huile de ricin éthoxylée et l'huile de ricin hydrogénée éthoxylée, par exemple le produit commercialisé sous la dénomination SIMULSOL™ 989 ; les compositions comprenant du stéarate de glycérol et de d'acide stéarique poly(éthoxylé) avec entre 5 moles et 150 moles d'oxyde d'éthylène, par exemple la composition comprenant de l'acide stéarique (éthoxylé) à 135 moles d'oxyde d'éthylène et du stéarate de glycérol commercialisée sous l'appellation SIMULSOL™ 165 ; les esters de sorbitan éthoxylés, par exemple les produits commercialisés sous la dénomination MONTANOX™ ; les esters de mannitan ; les esters de mannitan éthoxylés ; les esters de sucrose ; les esters de méthylglucoside.

Comme exemples de tensioactifs anioniques émulsionnants « cosmétiquement acceptables », on peut citer le décylphosphate, le cétylphosphate commercialisé sous l'appellation AMPHISOL™, le glycéryl stéarate citrate ; le cétéarylsulfate, la composition arachidyl/béhényl phosphates et arachidyl/béhényl alcools commercialisée sous l'appellation SENSANOV™ WR, le stéarate de sodium ou le stéarate de triéthanolammonium, les dérivés N-acylés d'acides aminés salifiés par exemple le stéaroyl glutamate. Comme exemples de tensioactifs cationiques émulsionnants « cosmétiquement acceptables », on peut citer les aminoxydes, le quaternium-82 et les tensioactifs décrits dans la demande internationale publiée sous le numéro WO 96/00719 et principalement ceux dont la chaîne grasse comprend au moins 16 atomes de carbone.

Comme exemples d'agents opacifiants et/ou nacrants « cosmétiquement acceptables », on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, et les alcools gras comportant de 12 à 22 atomes de carbone.

Comme exemples d'agents de texture « cosmétiquement acceptables », on peut citer des dérivés N-acylés d'acides aminés, par exemple la lauroyl lysine, l'octényl starch succinate et le myristyl polyglucoside commercialisés respectivement sous les appellations AMINOHOPE™ LL, DRYFLO™ et MONTANOV 14 ; les fibres de cellulose, les fibres de coton, les fibres de chitosane, le talc, la séricite, le mica.

Comme exemples de solvants et de co-solvants « cosmétiquement acceptables » seuls ou en mélanges, on peut citer l'eau, le glycérol, le diglycérol, les oligomères du glycérol, l'éthylène glycol, le propylène glycol, le butylène glycol, l'hexylène glycol, le diéthylène glycol, le xylitol, l'érythritol, le sorbitol et les alcools hydrosolubles tels que l'éthanol, l'isopropanol ou le butanol.

Comme exemples d'agents déodorants « cosmétiquement acceptables », on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le Triclosan™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'agents anti-oxydants « cosmétiquement acceptables », on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI : Tetrasodium Glutamate Diacetate).

Comme exemples de principes actifs optionnellement présents dans les émulsions (E), ($E_1$) et ($E_2$) telles que définies précédemment, il y a :

- les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ;

- les composés montrant une action apaisante notamment le SEPICALM™ S, l'allantoïne et le bisabolol ;
- les composés montrant une action hydratante comme l'urée, les hydroxyurées, le glycérolglucoside, le diglycérolglucoside, les polyglycérylglucosides, le glycérol, le diglycérol, le xylitylpolyglucoside commercialisé sous le nom de marque Aquaxyl™ ;
- les extraits végétaux riches en polyphénols comme les extraits de raisin, les extraits de pin, les extraits de vin, les extraits d'olives ;- les peptides N-acylés comme le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; - les extraits de blé par exemple la TENSINE™ ou la GLIADINE™ ; les extraits végétaux riches en tanins, les extraits végétaux riches en isoflavones ou les extraits végétaux riches en terpènes ;
- les extraits d'algues d'eau douce ou marines ; les extraits de plantes marines ; les extraits marins en général comme les coraux ; les cires essentielles ; les extraits bactériens ;
- les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ;
- les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™, le SURVICODE™ ;
- les actifs anti-photo vieillissement ; les actifs protecteurs de l'intégrité de la jonction dermo-épidermique ;
- les actifs augmentant la synthèse des composants de la matrice extracellulaire comme le collagène, les élastines, les glycosaminoglycanes ;
- les actifs agissant favorablement sur la communication cellulaire chimique comme les cytokines ou physiques comme les intégrines ;
- les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ;
- les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de centalla asiatica, de fucus, de romarin, de saule.

Les émulsions (E), ($E_1$) et ($E_2$) objet de la présente invention peuvent être préparées par une méthode connue de l'homme du métier qui comprend notamment les étapes suivantes :

- une étape de préparation de ladite phase aqueuse (A) en mélangeant l'ensemble des composants hydrophiles dans de l'eau ;
- une étape de préparation de ladite phase grasse phase (G) en mélangeant l'ensemble des composants lipophiles hydrophiles dans l'huile ou le mélange d'huiles et/ou la cire constitutive de ladite phase (G) puis en y ajoutant ladite composition (C) telle que définie précédemment ;
- une étape de mélange à la température appropriée, de ladite phase aqueuse (A) de ladite phase grasse phase (G) sous agitation mécanique avec un mobile de type ancre et à une vitesse de 60 tours/minute à une vitesse d'environ 100 tours/minute jusqu'à obtenir une émulsion homogène ; - optionnellement une étape d'ajustement du pH de l'émulsion.

Les émulsion (E), ($E_1$) et ($E_2$) peuvent être conditionnées sous forme pressurisée, par exemple dans un dispositif aérosol, dans un dispositif de type « flacon pompe » ou dans un dispositif muni d'une paroi ajourée par exemple une grille.
Selon un autre aspect plus particulier, l'invention a pour objet l'utilisation telle que définie précédemment, caractérisée en ce que ladite composition (C) est mise en œuvre au sein d'une formulation cosmétique (F) en comprenant de 1% à 10% massique et plus particulièrement de 2% à 5% massique, pour 100% de sa masse.
L'invention a aussi pour objet un procédé de séchage des cheveux caractérisé en ce qu'il comprend les étapes successives suivantes :

- au moins une étape a) d'application sur les cheveux d'une formulation cosmétique (F) comprenant pour 100% de sa masse de 0,2% à 40% massique d'une composition (C), ladite composition (C) comprenant pour 100% de sa masse,

  - de 10% massique à 90% massique d'au moins un polymère poly(farnésène) ayant une masse molaire moyenne en nombre supérieure ou égale 10.000 g/mol et inférieure ou égale à 120.000 g/mol, ledit polymère poly(farnésène) étant partiellement ou totalement hydrogéné et/ou fonctionnalisé avec des radicaux hydroxyle, et
  - de 10% massique à 90% massique d'un mélange (M) d'hydrocarbures dans lequel au moins 94% massique des dits hydrocarbures comportent de quinze à dix-neuf atomes de carbone,

- optionnellement, une étape b) au cours de laquelle à l'issue de l'étape a), les cheveux sont laissés au repos à température ambiante pendant une durée de 5 à 60 minutes puis
- une étape c) de séchage des cheveux à l'issue de l'étape a) ou optionnellement à l'issue de l'étape b), au moyen d'un appareil chauffant tel qu'un sèche-cheveux ou un casque chauffant.

A l'étape a) du procédé de séchage tel que défini ci-dessus, ladite formulation (F) est appliquée de façon directe ou indirecte, par exemple au moyen de tout type de support destiné à être mis en contact avec la peau ou les cheveux comme le papier, les lingettes ou le textile.
Par « température ambiante », on entend à l'étape b) du procédé de séchage tel que défini ci-dessus, une température comprise entre 15°C et 35°C, notamment entre 20°C et 30°C.
Selon un aspect particulier du procédé tel que défini ci-dessus, ledit mélange (M) d'hydrocarbures présent dans la composition (C) comprise dans la formulation (F) mise en œuvre à l'étape a), est un mélange d'hydrocarbures saturés comprenant, pour 100% de sa masse :

- de 15% à 20% massique d'alcanes (linéaires, iso-alcanes et cyclo-alcanes) comportant 17 atomes de carbone,
- de 70% à 75% massique d'alcanes (linéaires, iso-alcanes et cyclo-alcanes) comportant 18 atomes de carbone, et
- de 4% à 6% massique d'alcanes (linéaires, iso-alcanes et cyclo-alcanes) comportant 19 atomes de carbone.

Selon un aspect plus particulier du procédé tel que défini ci-dessus, ledit mélange (M) d'hydrocarbures présent dans la composition (C) comprise dans la formulation (F) mise en œuvre à l'étape a), est un mélange d'hydrocarbures saturés comprenant, pour 100% de sa masse, :

- 3,8% d'alcanes linéaires comportant de quinze à dix-neuf atomes de carbone,
- 96,2% d'iso-alcanes comportant de quinze à dix-neuf atomes de carbone, et
- moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

Selon un autre aspect particulier du procédé tel que défini ci-dessus, ledit polymère poly(farnésène) présent dans la composition (C) comprise dans la formulation (F) mise en œuvre à l'étape a), a une masse molaire moyenne en nombre supérieure ou égale 20.000 g/mol et inférieure ou égale à 90.000 g/mol, et en particulier une masse molaire moyenne en nombre supérieure ou égale 40.000 g/mol et inférieure ou égale à 80.000 g/mol ; ledit polymère poly(farnésène) présent dans ladite composition (C) comprise dans la formulation (F) mise en œuvre à l'étape a), est plus particulièrement totalement hydrogéné, fonctionnalisé avec des radicaux hydroxyle et a une masse molaire moyenne en nombre égale à 71.000 g/mole.
Selon un aspect tout particulier du procédé tel que défini ci-dessus, ladite composition (C) comprise dans la formulation (F) mise en œuvre à l'étape a), comprend pour 100% de sa masse,

- 40% massique d'un polymère poly(farnésène) totalement hydrogéné, fonctionnalisé avec des groupes hydroxyle et ayant une masse molaire moyenne en nombre égale à 71.000, et
- 60% massique d'un mélange (M) un mélange d'hydrocarbures saturés comprenant, pour 100% de sa masse :

  - 3,8% d'alcanes linéaires comportant de quinze à dix-neuf atomes de carbone,
  - 96,2% d'iso-alcanes comportant de quinze à dix-neuf atomes de carbone, et
  - moins de 0,1% de cycloalcanes comportant de quinze à dix-neuf atomes de carbone,

  et dans lequel :

  - 17,1% massique d'alcanes (linéaires, iso-alcanes et cyclo-alcanes) comportent 17 atomes de carbone,
  - 72,5% massique d'alcanes (linéaires, iso-alcanes et cyclo-alcanes) comportent 18 atomes de carbone, et
  - 4,7% massique d'alcanes (linéaires, iso-alcanes et cyclo-alcanes) comportent 19 atomes de carbone.

Selon un autre aspect particulier du procédé tel que défini ci-dessus, ladite composition (C) est mise en œuvre au sein d'une émulsion (E) d'une phase aqueuse (A) et d'une phase grasse (G) comprenant pour 100% de sa masse :

- de 50% à 98% massique, de ladite phase aqueuse (A), et
- de 2% à 50% massique, de ladite phase grasse (G),

ladite phase grasse (G) comprenant, pour 100% de sa masse :

- de 10% à 80% massique, de ladite composition (C),
- de 0,5% à 20% massique d'un agent tensioactif émulsionnant (S) ou d'un mélange d'agents tensioactifs émulsionnants ($M_S$) du type huile-dans-eau ou du type eau-dans huile, et
- de 0% à 89,5% massique, d'une huile ou de plusieurs huiles et/ou d'une cire, ladite cire étant différente de ladite composition (C).

Selon un autre aspect particulier du procédé tel que défini ci-dessus, ladite composition (C) est mise en œuvre au sein d'une formulation cosmétique (F) en comprenant pour 100% de sa masse, de 1% à 10% massique et plus particulièrement de 2% à 5% massique.

L'invention a aussi pour objet un procédé de mise en forme des cheveux caractérisé en ce qu'il comprend les étapes successives suivantes :

- une étape d) d'application sur les cheveux secs ou humides, d'une formulation cosmétique (F) comprenant pour 100% de sa masse 0,2% à 40% massique d'une composition (C) comprenant pour 100% de sa masse,
  - de 10% massique à 90% massique d'au moins un polymère poly(farnésène) ayant une masse molaire moyenne en nombre supérieure ou égale 10.000 g/mol et inférieure ou égale à 120.000 g/mol, ledit polymère poly(farnésène) étant partiellement ou totalement hydrogéné et/ou fonctionnalisé avec des radicaux hydroxyle, et
  - de 10% massique à 90% massique d'un mélange (M) d'hydrocarbures dans lequel au moins 94% massique des dits hydrocarbures comportent de quinze à dix-neuf atomes de carbone.
- optionnellement, une étape e) au cours de laquelle à l'issue de l'étape d), les cheveux sont laissés au repos à température ambiante pendant une durée de 5 à 60 minutes puis,
- une étape f) de mise en forme des cheveux à l'issue de l'étape d) ou optionnellement à l'issue de l'étape e), au moyen d'un appareil chauffant, tel qu'un fer à lisser ou un fer à boucler.

Par mise en forme des cheveux, on désigne dans la définition du procédé tel que défini ci-dessus, les actions consistant à peigner, à lisser, à faire onduler et/ou à faire boucler les cheveux.

Par « température ambiante », on entend dans le procédé de mise en forme tel que défini ci-dessus, une température comprise entre 15°C et 35°C, notamment entre 20°C et 30°C.

A l'étape d) du procédé de mise en forme tel que défini ci-dessus, ladite émulsion (F) est appliquée de façon directe ou indirecte, par exemple au moyen de tout type de support destiné à être mis en contact avec la peau ou les cheveux comme le papier, les lingettes ou le textile.

Selon un aspect plus particulier du procédé tel que défini ci-dessus, ledit mélange (M) d'hydrocarbures présent dans la composition (C) comprise dans la formulation (F) mise en œuvre à l'étape a), est un mélange d'hydrocarbures saturés comprenant, pour 100% de sa masse, :

- 3,8% d'alcanes linéaires comportant de quinze à dix-neuf atomes de carbone,
- 96,2% d'iso-alcanes comportant de quinze à dix-neuf atomes de carbone, et
- moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

Selon un autre aspect particulier du procédé tel que défini ci-dessus, ledit polymère poly(farnésène) présent dans la composition (C) comprise dans la formulation (F) mise en œuvre à l'étape a), a une masse molaire moyenne en nombre supérieure ou égale 20.000 g/mol et inférieure ou égale à 90.000 g/mol, et en particulier une masse molaire moyenne en nombre supérieure ou égale 40.000 g/mol et inférieure ou égale à 80.000 g/mol ; ledit polymère poly(farnésène) présent dans ladite composition (C) comprise dans la formulation (F) mise en œuvre à l'étape a), est plus

particulièrement totalement hydrogéné, fonctionnalisé avec des radicaux hydroxyle et a une masse molaire moyenne en nombre égale à 71.000 g/mole.

Selon un aspect tout particulier du procédé tel que défini ci-dessus, ladite composition (C) comprise dans la formulation (F) mise en œuvre à l'étape a), comprend pour 100% de sa masse,

- 40% massique d'un polymère poly(farnésène) totalement hydrogéné, fonctionnalisé avec des groupes hydroxyle et ayant une masse molaire moyenne en nombre égale à 71.000, et
- 60% massique d'un mélange (M) un mélange d'hydrocarbures saturés comprenant, pour 100% de sa masse :
  - 3,8% d'alcanes linéaires comportant de quinze à dix-neuf atomes de carbone,
  - 96,2% d'iso-alcanes comportant de quinze à dix-neuf atomes de carbone, et
  - moins de 0,1% de cycloalcanes comportant de quinze à dix-neuf atomes de carbone,

  et dans lequel :

  - 17,1% massique d'alcanes (linéaires, iso-alcanes et cyclo-alcanes) comportent 17 atomes de carbone,
  - 72,5% massique d'alcanes (linéaires, iso-alcanes et cyclo-alcanes) comportent 18 atomes de carbone, et
  - 4,7% massique d'alcanes (linéaires, iso-alcanes et cyclo-alcanes) comportent 19 atomes de carbone.

Selon un autre aspect particulier du procédé tel que défini ci-dessus, ladite composition (C) est mise en œuvre au sein d'une émulsion (E) d'une phase aqueuse (A) et d'une phase grasse (G) comprenant pour 100% de sa masse :

- de 50% à 98% massique, de ladite phase aqueuse (A), et
- de 2% à 50% massique, de ladite phase grasse (G),

ladite phase grasse (G) comprenant, pour 100% de sa masse :

- de 10% à 80% massique, de ladite composition (C),
- de 0,5% à 20% massique d'un agent tensioactif émulsionnant (S) ou d'un mélange d'agents tensioactifs émulsionnants ($M_S$) du type huile-dans-eau ou du type eau-dans huile, et
- de 0% à 89,5% massique, d'une huile ou de plusieurs huiles et/ou d'une cire, ladite cire étant différente de ladite composition (C).

Selon un autre aspect particulier du procédé tel que défini ci-dessus, ladite composition (C) est mise en œuvre au sein d'une formulation cosmétique (F) en comprenant pour 100% de sa masse, de 1% à 10% massique et plus particulièrement de 2% à 5% massique.

## Exemples expérimentaux

### Préparation de la composition (C) mise en œuvre dans les exemples

Le polymère (poly)farnésène compris dans la composition (C) mise en œuvre dans les exemples suivants est totalement hydrogéné et fonctionnalisé avec des radicaux hydroxyle ; il a une masse molaire moyenne en nombre de 71 000 g/mol. Il a été préparé selon le mode opératoire décrit dans WO2018/052709. Le mélange (M) compris dans cette même composition (C) est disponible dans le commerce sous le nom de marque Emogreen™ L19 ; il comprend pour 100% de sa masse 3,8% d'alcanes linéaires comportant de quinze à dix-neuf atomes de carbone, 96,2 % d'iso-alcanes comportant de quinze à dix-neuf atomes de carbone, et une proportion massique inférieure à 0,1% de cyclo-alcanes comportant de quinze à dix-neuf atomes de carbone ; parmi ces constituants pour 100% de sa masse, 17,1% massique sont des alcanes (linéaires, iso-alcanes et cyclo-alcanes) comportant 17 atomes de carbone, 72,5% massique sont des alcanes (linéaires, iso-alcanes et cyclo-alcanes) comportant 18 atomes de carbone et 4,7% massique sont des alcanes (linéaires, iso-alcanes et cyclo-alcanes) comportant 19 atomes de carbone. Le mélange (M) a été préparé selon le mode opératoire décrit dans WO 2020/144440 A1.

La quantité désirée en Emogreen™L19 est versée dans un réacteur en verre muni d'une double enveloppe dans laquelle circule un fluide thermostaté et d'une agitation mécanique munie d'un agitateur équipé d'une pâle de type ancre. La vitesse d'agitation est réglée à 50 tours/minute et la température à 25°C. La quantité désirée de polymère poly(farnésène) est ensuite introduite sous agitation modérée à 60°C. Les quantités introduites sont telles qu'Emogreen™L19 représente une teneur massique de 60% et le polymère poly(farnésène) représente une teneur massique de 40% pour 100% massique de la composition (C) ainsi obtenue.

**Mise en évidence de la compatibilité de la composition (C) dans des formulations destinées à être utilisées par application sur les cheveux.**

**A. Compatibilité de la composition (C) dans des formulations de conditionnement des cheveux comprenant un agent conditionneur cationique.**

Préparation de formulations : On prépare les formulations selon l'invention ($F_1$) et ($F_2$) et les formulations comparatives ($F'_1$) et ($F'_2$), dont les compositions sont consignées dans le tableau 1 suivant, les proportions étant exprimées en pourcentages massiques.

Evaluation des formulations : On introduit dans une enceinte climatique régulée à 20°C une quantité de 100 $cm^3$ de la composition à tester et contenue dans un flacon de 250 $cm^3$, pendant une durée de 3 mois. On évalue après une durée de 7 jours et après une durée de 3 mois l'aspect visuel de la composition testée. On réitère la même opération avec de nouveaux échantillons des formulations en régulant la température intérieure de l'enceinte climatique à 45°C On évalue après une durée de 3 mois l'aspect visuel de la composition testée. Les résultats obtenus sont consignés dans le tableau 2 suivant.

[Tableau 1]

| | Proportions massiques des constituants pour chacune des formulations | | | |
|---|---|---|---|---|
| | ($F_1$) | ($F_2$) | ($F'_1$) | ($F'_2$) |
| Eau | qsp 100% | qsp 100% | qsp 100% | qsp 100% |
| Dissolvine™ GL47 | 0,30% | 0,30% | 0,30% | 0,30% |
| Glycérine | 3,00% | 3,00% | 3,00% | 3,00% |
| AG $C_{16}$-$C_{18}$ | 4,00% | 4,00% | 4,00% | 4,00% |
| Varisoft™ BT85 | 3,00% | 3,00% | 3,00% | 3,00% |
| DL alpha tocophérol | 0,05% | 0,05% | 0,05% | 0,05% |
| Huile de coco | 2,00% | 2,00% | 2,00% | 2,00% |
| Lanol™ 2681 | 3,00% | 3,00% | 3,00% | 3,00% |
| Natrosol™250HPC | 0,80% | 0,80% | 0,80% | 0,80% |
| Composition (C) | 3,00% | 5,00% | 0% | 0% |
| Massocare™ SIL CPD | 0% | 0% | 3,00% | 5,00% |
| Euxyl™ PE9010 | 1,00% | 1,00% | 1,00% | 1,00% |
| Acide citrique | qs pH 4.5 | qs pH 4.5 | qs pH 4.5 | qs pH 4.5 |

[Tableau 2]

| | Aspect des formulations | | | |
|---|---|---|---|---|
| | ($F_1$) | ($F_2$) | ($F'_1$) | ($F'_2$) |
| Après 7 jours à 20°C | Homogène | Homogène | Homogène | Homogène |
| Après 3 mois à 20°C | Homogène | Homogène | Homogène | Homogène |
| Après 3 mois à 45°C | Homogène | Homogène | Exsudation | Exsudation |
| | Viscosité à 20°C (BKF RVT M4V6) | | | |
| | ($F_1$) | ($F_2$) | ($F'_1$) | ($F'_2$) |
| Après 7 jours à 20°C | 62.000 mPa.s | 76.000 mPa.s | 66.000 mPa.s | 78.000 mPa.s |
| Après 3 mois à 20°C | 68.000 mPa.s | 77.000 mPa.s | 70.000 mPa.s | 68.000 mPa.s |

**B. Compatibilité d'une composition (C) dans des émulsions de conditionnement des cheveux de type huile-dans-eau des cheveux comprenant un polyélectrolyte cationique réticulé.**

Préparation de formulations : On prépare les formulations ($F_3$) et ($F_4$) selon l'invention et les ($F'_3$) et ($F'_4$) formulations comparatives, dont les compositions sont consignées dans le tableau 3 suivant, les proportions étant exprimées en pourcentages massiques.

Evaluation des formulations : On évalue les formulations ($F_3$), ($F_4$), ($F'_3$) et ($F'_4$) selon le même protocole que celui décrit dans la partie « Evaluation des formulations » du paragraphe A précédent. Les résultats obtenus sont consignés dans le tableau 4 suivant.

[Tableau 3]

| | Proportions massiques des constituants pour chacune des formulations | | | |
|---|---|---|---|---|
| | ($F_3$) | ($F_4$) | ($F'_3$) | ($F'_4$) |
| Eau | qsp 100% | qsp 100% | qsp 100% | qsp 100% |
| SOLAGUM™ Tara | 0,30% | 0,30% | 0,30% | 0,30% |
| Glycérine | 2,00% | 2,00% | 2,00% | 2,00% |
| Huile d'argan | 2,00% | 2,00% | 2,00% | 2,00% |
| DL alpha tocophérol | 0,50% | 0,50% | 0,50% | 0,50% |
| Simulquat™ HC305 | 3,00% | 3,00% | 3,00% | 3,00% |
| Composition (C) | 3,00% | 5,00% | 0% | 0% |
| Massocare™ SIL CPD | 0% | 0% | 3,00% | 5,00% |
| Euxyl PE9010 | 1,00% | 1,00% | 1,00% | 1,00% |
| Acide citrique | qs pH 4.5 | qs pH 4.5 | qs pH 4.5 | qs pH 4.5 |

[Tableau 4]

| | Aspect des formulations | | | |
|---|---|---|---|---|
| | ($F_3$) | ($F_4$) | ($F'_3$) | ($F'_4$) |
| Aspect après 7 jours à 20°C | Homogène | Homogène | Homogène | Homogène |
| Aspect après 3 mois à 20°C | Homogène | Homogène | Homogène | Homogène |
| Après 3 mois à 45°C | Homogène | Homogène | Homogène | Exsudation |
| | Viscosité à 20°C (BKF RVT M4V6) | | | |
| | ($F_3$) | ($F_4$) | ($F'_3$) | ($F'_4$) |
| Après 7 jours à 20°C | 167.000 mPa.s | 105.000 mPa.s | 180.000 mPa.s | 104.000 mPa.s |
| Après 3 mois à 20°C | 157.000 mPa.s | 95.000 mPa.s | 157.000 mPa.s | 75.000 mPa.s |

**C. Compatibilité d'une composition (C) dans des émulsions non rincées de type huile-dans-eau de conditionnement des cheveux comprenant au moins un agent tensioactif, et destinées à être appliquées sur les cheveux.**

Préparation de formulations : On prépare les formulations ($F_5$) et ($F_6$) selon l'invention et les ($F'_5$) et ($F'_6$) formulations comparatives, dont les compositions sont consignées dans le tableau 5 suivant, les proportions étant exprimées en pourcentages massiques.

[Tableau 5]

| | Proportions massiques des constituants pour chacune des formulations | | | |
|---|---|---|---|---|
| | ($F_5$) | ($F_6$) | ($F'_5$) | ($F'_6$) |
| Eau | qsp 100% | qsp 100% | qsp 100% | qsp 100% |

(suite)

| | Proportions massiques des constituants pour chacune des formulations | | | |
|---|---|---|---|---|
| | ($F_5$) | ($F_6$) | ($F'_5$) | ($F'_6$) |
| Dissolvine GL47 | 0,30% | 0,30% | 0,30% | 0,3% |
| Sepimax™ Zen) | 0,60% | 0,60% | 0,60% | 0,60% |
| Fluidifeel™ Easy | 3,00% | 3,00% | 3,00% | 3,00% |
| Huile d'argan | 3,00% | 2,00% | 3,00% | 2,00% |
| DL alpha tocophérol | 0,050% | 0,05% | 0,05% | 0,05% |
| Composition (C) | 2,00% | 3,00% | 0% | 0% |
| Massocare™ SIL CPD | 0% | 0% | 2,00% | 3,00% |
| Euxyl™ PE9010 | 1,00% | 1,00% | 1,00% | 1,00% |
| Acide citrique | qs pH 4.5 | qs pH 4.5 | qs pH 4.5 | qs pH 4.5 |

Evaluation des formulations : Les formulations ($F_5$), ($F_6$), ($F'_5$) et ($F'_6$) sont évaluées selon le même protocole que précédemment. Les résultats sont consignés dans le tableau 6 suivant.

[Tableau 6]

| | Aspect des formulations | | | |
|---|---|---|---|---|
| | ($F_5$) | ($F_6$) | ($F'_5$) | ($F'_6$) |
| Après 7 jours à 20°C | Homogène, liquide lisse | Homogène, liquide lisse | Hétérogène, liquide lisse avec globules | Hétérogène, liquide lisse avec globules |
| Après 3 mois à 20°C | Homogène, liquide lisse | Homogène, liquide lisse | Hétérogène, liquide lisse avec globules | Hétérogène, liquide lisse avec globules |
| Après 3 mois à 45°C | Homogène, liquide lisse | Homogène, liquide lisse | Hétérogène, liquide lisse avec globules | Hétérogène, liquide lisse avec globules |
| | Viscosité à 20°C (BKF RVT M2V6) : | | | |
| | ($F_5$) | ($F_6$) | ($F'_5$) | ($F'_6$) |
| Après 7 jours à 20°C | 2.440 mPa.s | 2.620 mPa.s | 1.540 mPa.s | 2480 mPa.s |
| Après 3 mois à 20°C | 2.420 mPa.s | 2.155 mPa.s | 1.720 mPa.s | 2.200 mPa.s |

**D. Compatibilité d'une composition (C) dans des huiles pour application sur les cheveux**

Préparation de formulations : On prépare la formulation selon l'invention ($F_7$) et les formulations comparatives ($F'_7$) et ($F'_8$), dont les compositions sont consignées dans le tableau 7 suivant, les proportions étant exprimées en pourcentages massiques.

[Tableau 7]

| | Proportions massiques des constituants pour chacune des formulations | | |
|---|---|---|---|
| | ($F_7$) | ($F'_8$) | ($F'_7$) |
| Composition (C) | 50% | / | / |
| Emogreen L19 | / | / | 50% |
| Massocare SIL CPD | / | 50% | / |
| Huile argan | 2,50% | 2,50% | 2,50% |

(suite)

| | Proportions massiques des constituants pour chacune des formulations | | |
|---|---|---|---|
| | ($F_7$) | ($F'_8$) | ($F'_7$) |
| Huile coco | 2,50% | 2,50% | 2,50% |
| Triglycérides 5545 | 44,80% | 44,80% | 44,80% |
| DL alpha tocophérol | 0,20% | 0,20% | 0,20% |

Evaluation des formulations : On évalue les formulations ($F_7$), ($F'_7$), ($F'_8$) selon le même protocole que précédemment. Les résultats sont consignés dans le tableau 8 suivant.

[Tableau 8]

| | Aspect des formulations | | |
|---|---|---|---|
| | ($F_7$) | ($F'_8$) | ($F'_7$) |
| Après 7 jours à 20°C | Homogène | Hétérogène, non miscible | Homogène |
| Après 3 mois à 20°C | Homogène | Hétérogène, non miscible | Homogène |
| Après 3 mois à 45°C | Homogène | Hétérogène, non miscible | Homogène |
| | Viscosité à 20°C (BKF LVT M2) : | | |
| | ($F_7$) | ($F'_8$) | ($F'_7$) |
| | 120 mPa.s | No mesurable | < 50 mPa.s |

**E. Observations et conclusions**

Les résultats obtenus aux paragraphes précédents permettent de conclure ce qui suit :

- Les formulation ($F_1$) et ($F_2$) selon l'invention comprenant un agent conditionneur et la composition (C) présentent un aspect homogène après un stockage d'une durée de 3 mois à 45°C, ce qui n'est pas observé pour les formulations ($F'_1$) et ($F'_2$) comprenant les dérivés siliconés.
- L'émulsion de type E/H (formulation ($F_4$)) selon l'invention comprenant un polyélectrolyte cationique réticulé, et 5% de la composition (C) présente un aspect homogène après un stockage d'une durée de 3 mois à 45°C, alors que la formulation ($F'_4$) comprenant des dérivés siliconés exsude.
- Les émulsions de type H/E non rincées de conditionnement des cheveux comprenant au moins un agent tensioactif, et la composition (C) ont un aspect plus homogène que les émulsions comparatives (formulations ($F'_5$ et $F'_6$)) qui comportent des globules.
- La formulation ($F_7$) comprenant la composition (C) présente un aspect homogène après un stockage d'une durée de 3 mois à 45°C, ce qui n'est pas observé avec la formulation ($F'_8$) qui présente un aspect hétérogène.

Nous pouvons donc conclure de l'ensemble de ces résultats, une très bonne compatibilité des formulations comprenant la composition (C) avec des schémas de formulation destinés à des applications dans le soin et/ou l'hygiène des cheveux.

**Mise en évidence de la préservation de l'intégrité de la kératine du cortex du cheveu d'une formulation comprenant la composition (C).**

L'évaluation de l'effet de ladite composition (C) pour préserver l'intégrité de la kératine du cortex du cheveu, est effectuée par la technologie Xpolar® développée par la société Kmax Innovative System. A.

**A. Principe de la méthode d'évaluation**

La technologie Xpolar® est un mode d'imagerie intégrée sur une plateforme de microscopie, permettant d'observer et de mesurer les modifications polarimétriques produites par les échantillons étudiés. Elle apporte de l'information quant à la biréfringence de l'échantillon évalué. Par "biréfringence" on entend la propriété physique d'un

matériau dans lequel la lumière se propage de façon anisotrope. Dans un milieu biréfringent, l'indice de réfraction n'est pas unique, il dépend de la direction de polarisation de l'onde lumineuse. Le paramètre « k-index », mesuré par la technologie Xpolar® apporte de l'information sur la biréfringence de l'échantillon en chaque point de l'image, il peut être utilisé de façon quantitative pour caractériser l'échantillon. Le cheveu est composé de kératines, une famille de protéines fibreuses. Les fibres de kératines comportent une partie cristalline liée par des forces de Coulomb et des interactions hydrophobes. Cette structure cristalline des fibres de kératine du cortex confère au cheveu la capacité de modifier la polarisation de la lumière qui le traverse, au travers d'une propriété appelée biréfringence. Un cheveu sain (contrôle), à savoir un cheveu n'ayant pas été exposé à une intense chaleur, présente une valeur k-index élevée. La coloration à l'intérieur de la fibre est homogène et est composée des couleurs situées au niveau maximal de la mesure (coloration rouge orangé dominent). Lorsque les fibres de la kératine à l'intérieur du cheveu ne sont pas altérées, notamment par l'exposition à une chaleur intense, elles sont organisées en réseau cristallin, et cela se traduit par une valeur de « k-index élevé ». A contrario, l'exposition du cheveu à un stress thermique, comme l'exposition à une chaleur intense modifier la structure interne de la kératine, entraînant une désorganisation et une modification des propriétés optiques de la kératine, se traduisant in fine par la diminution du « k-index » et par une forte diminution de la coloration à l'intérieur de la fibre qui devient hétérogène et avec des teintes différentes (vert, bleu, violet).

### B. Mode opératoire de la méthode d'évaluation

La composition à tester est appliquée sur des cheveux secs avec un peigne comprenant environ soit 30 mg de ladite composition à tester. Les cheveux sont ensuite massés pendant 30 secondes, puis séchés à température ambiante (20°C) pendant 30 minutes. Le traitement thermique est ensuite effectué avec un fer à lisser à une température de 210°C pendant 50 passages. Les cheveux ainsi traités sont laissés à température ambiante pendant 30 minutes.

On prépare les lames d'observation en enrobant des cheveux dans un milieu de congélation, puis en congelant (-20°C) des cheveux enrobés, puis en réalisant une coupe de 30 μm puis un montage des lames sur l'appareil d'observation (appareil Xpolar, Grossissement: ×20 et acquisition de 25 images par condition).

### C. Compositions évaluées

Trois compositions et quatre formules identifiées respectivement dans les tableaux 9 et 10 ont été évaluées.

[Tableau 9]

| Références | LCE21076 | LCE21077 | LCE21078 |
|---|---|---|---|
| Ingrédient | Composition (C) | Massocare™ SIL DMD | Emogreen™ L19 |
| Aspect | Liquide incolore | Liquide incolore | Liquide incolore |

[Tableau 10]

| Références | LCE21071 | LCE21072 | LCE21073 | LCE21075 |
|---|---|---|---|---|
| Eau déminéralisée | qs 100% | | | |
| Dissolvine™ GL47 | 0,30% | | | |
| Sepimax™ Zen | 0,60% | | | |
| Fluidifeel™ Easy | 3,00% | | | |
| Huile d'argan | 2,00% | | | |
| DL alpha tocophérol | 0,05% | | | |
| Composition (C1) | 3,00% | 0% | 0% | 0% |
| Massocare™ SIL DMD | 0% | 3,00% | 0% | 0% |
| Emogreen™ L19 | 0% | 0% | 0% | 3,00% |
| Euxyl™ PE9010 | 1,00% | | | |
| Acide lactique | qs pH = 4.5 - 5.0 | | | |

De plus, la même évaluation a été effectuée avec un cheveu non traité n'ayant pas subi de traitement thermique ("contrôle négatif"), et avec un cheveu non traité mais ayant subi le traitement thermique décrit dans le mode opératoire ci-dessus ("contrôle positif").

**D. Résultats obtenus**

Les valeurs de « k-index » obtenues sont consignées dans le tableau 11 ci-dessous. Les résultats sont exprimés par deux valeurs :

La variation par rapport au contrôle négatif $VA_1$ calculée comme suit : $VA_1$ = [(k-index$_i$ - k-index $_{\text{contrôle négatif}}$) x 100 / k-index $_{\text{contrôle négatif}}$

Le pourcentage de protection $VA_2$ calculé comme suit : $VA_2$ = 100 - [(k-index$_i$ - k-index $_{\text{contrôle négatif}}$) x 100 / (k-index $_{\text{contrôle négatif}}$ - k-index $_{\text{contrôle positif}}$)]

[Tableau 11]

| Composition testée | k-index (x $10^{-4}$) | $VA_1$ | $VA_2$ |
|---|---|---|---|
| Contrôle négatif | 87.82 ± 16.13 | | |
| Contrôle positif | 58.42 ± 9.70 | -33% | |
| LCE 21076 | 69.65 ± 9.70 | -21% | 38,2% |
| LCE 21077 | 68.16 ± 13.45 | -22% | 33,1% |
| LCE 21078 | 51.83 ± 6.56 | -41% | - 22,4% |
| LCE 21071 | 60.64 ± 11.65 | -31% | 22,0% |
| LCE 21072 | 67.24 ± 11.81 | -23% | 40,9% |
| LCE 21073 | 52.96 ± 8.11 | -40% | na |
| LCE 21075 | 62.35 ± 8.88 | -29% | 26,9% |

Une analyse statistique complémentaire a été réalisée entre les différentes compositions montré les différences significatives exprimées dans le tableau 12 ci-après :

[Tableau 12]

| Conditions étudiées | Différence significative (p) |
|---|---|
| LCE 21073/LCE 21071 | Oui (p=9 $10^{-3}$) |
| LCE 21073/ LCE 21072 | Oui (p=3.5 $10^{-5}$) |
| LCE 21073/ LCE 21075 | Oui (p=2.8 $10^{-4}$) |
| LCE 21071/ LCE 21072 | Non (p=8 $10^{-2}$) |
| LCE 21076/ LCE 21077 | Non (p=0.72) |
| LCE 21076/ LCE 21078 | Oui (p=1.14 $10^{-5}$) |

Observations et commentaires : La composition (C) (LCE21076) et la composition (LCE21077) à base de dérivés siliconés, montrent un pourcentage de protection à l'exposition thermique du fer à lisser respectivement de 38,2% et de 33,1% ; la composition (LCE21078) à base du mélange d'hydrocarbure Emogreen™L19 montre un pourcentage de protection négatif, démontrant ainsi son inefficacité et une observation visuelle lors de l'expérimentation a souligné un fort état dégradé de la kératine. Parmi les formulations testées, pour la formulation (LCE21073) dite "placebo" car ne comprenant ni la composition (C) ni des dérivés siliconés ni autre agents filmogènes ou protecteurs de la kératine, on observe une altération de la kératine provoquée par le traitement thermique. Les résultats des pourcentages de protection montrent que les 3 formulations (LCE21071) (LCE21072) (LCE21075) apportent une protection sur la kératine qui est significativement différente à celle apportée par la formule placebo (LCE21073). La composition (C), montre une action protectrice sur la kératine du cortex du cheveu en réduisant de 38% l'effet de la chaleur sur l'intégrité de la kératine. Cette propriété est vérifiée en formulation versus placebo ou le taux de protection de la kératine est de 22%. Les formulations selon l'invention comprenant la composition (C) sont donc des solutions alternatives aux dérivés siliconés, par exemple

les polydiméthylsiloxane ou PDMS ou diméthicone), et/ou les diméthiconols, pour conférer à des formulations cosmétiques des propriétés permettant de ralentir ou d'empêcher l'apparition d'effets inesthétiques sur les cheveux humains suite à l'exposition à une chaleur intense. Cette alternative met en œuvre des compositions chimiques d'origines végétales et/ou biodégradables.

**Exemples formulations cosmétiques**

**Emulsion eau dans huile pour soin capillaire avant séchage**

| Composition de l'émulsion | Proportions massiques des constituants |
|---|---|
| Eau | qsp 100% |
| Glycérine | 2,00% |
| Simulquat™ HC 305(40% matière active) | 2,50% (soit 1% matière active) |
| Triéthanolamine 6% | Jusqu'à pH = 4,0 - 4,5 |
| Easynov™ | 2,50% |
| Composition (C) | 10,00% |
| Euxyl™ PE 9010 | 1,00% |

**Baume capillaire de type émulsion huile dans eau**

| Composition du baume | Proportions massiques des constituants |
|---|---|
| Eau déminéralisée | Qsp |
| Dissolvine™ GL47S | 0,30% |
| Sepimax™ Zen | 0,60% |
| Solagum™ AX | 0,50% |
| Fluidifeel™ Easy | 0,30% |
| Huile d'argan | 2,00% |
| DL alpha tocophérol | 0,05% |
| Composition (C) | 3,00% |
| Euxyl™ PE9010 | 1,00% |
| Acide lactique | Jusqu'à pH=4,5 |

**Masque capillaire sublime**

| Composition du masque | Proportions massiques des constituants |
|---|---|
| Aqua/Eau | Qsp 100% |
| Glycérol | 2,00% |
| SOLAGUM™ TARA | 0,30% |
| Composition (C) | 3,00% |
| Cocos Nucifera Oil | 1,00% |

(suite)

| Composition du masque | Proportions massiques des constituants |
|---|---|
| Moringa Oleifera Seed Oil | 1,00% |
| SIMULQUAT™ HC 305 | 3,50% |
| XYLISHINE™ | 3,00% |
| parfum | 0,20% |
| Phenoxyethanol - Ethylhexylglycerin | 1,00% |
| Colorant | 0,08% |

**Huile capillaire précieuse**

| Composition de l'huile | Proportions massiques des constituants |
|---|---|
| LANOL™ 2681 | 48,60% |
| Composition (C) | 5,00% |
| Limnanthes Alba (Meadowfoam) Seed Oil | 5,00% |
| Cocos Nucifera Oil | 20,00% |
| Simmondsia Shinensis Seed Oil | 20,00% |
| Fragrance | 0,20% |
| Tocophérol | 0,20% |
| SEA SATIN™ | 1,00% |

Sérum lissant « perfecteur » pour la protection des cheveux contre la chaleur, la douceur et la souplesse

| Composition du sérum | Proportions massiques des constituants |
|---|---|
| Aqua/Eau | Qsp 100% |
| Tetrasodium Glutamate Diacetate | 0,30% |
| SEPIMAX™ ZEN | 0.60% |
| SOLAGUM™ TARA | 0,50% |
| FLUIDIFEEL™ EASY | 3,00% |
| Composition (C) | 3,00% |
| Argania Spinosa Kernel Oil | 2,00% |
| Isopropyl myristate | 1,00% |
| ALARIANE™ | 1,00% |
| XYLISHINE™ | 3,00% |
| Tocophérol | 0,05% |
| parfum | 0,20% |
| Phenoxyethanol - Ethylhexylglycerin | 1,00% |
| Acide lactique | 0,25% |

Les composés identifiés par leur nom commercial dans les différentes formulations ainsi que dans l'exemple expérimental sont explicités ci-après :

Varisoft™ BT85 est un agent antistatique et conditionneur, identifié sous le nom INCI : Behentrimonium Chloride ;)
AG $C_{16}$-$C_{18}$ est un mélange d'alcools cétylique et stéarylique (ratio massique = 50/50) ;
Natrosol™ 250HPC un nom commercial de l'hydroxyéthylcellulose ;
Massocare™ SIL CPD est un agent émolliant, identifié par le nom INCI : Cyclopentasiloxane (and) PEG/PPG-18/18 Dimethicone) ;
SIMULQUAT™ HC305 est un agent épaississant et stabilisant d'émulsions identifié sous le nom INCI : Acrylamidopropyltrimonium Chloride/Acrylates Copolymer (and) Isoliexadecane (and) Coceth-7) ;
Easynov™ est un agent émulsionnant de type eau-dans-huile identifié sous le nom INCI : octyldodecanol octyldodecyl xyloside and peg30 dipolyhydroxystearate) ;
Euxyl™ PE9010 est un agent conservateur identifié sous le nom INCI : phenoxyethanol and ethylhexylglycerin) ;
Dissolvine™ GL47 est un agent séquestrant, identifié sous le nom INCI : Tetrasodium Glutamate Diacetate) ;
SEPIMAX™Zen est un agent épaississant polymérique, utilisé comme modificateur de rhéologie de formulations cosmétiques, dont le squelette polymérique est constitué par les unités monomériques issues de l'acide 2-méthyl 2-[(1-oxo 2-propènyl) amino] 1-propanesulfonique sous forme sodique, de N,N-diméthyl acrylamide, de méthacrylate de lauryle tétraéthoxylé et comprend le triméthylol propanetriacrylate comme agent de réticulation, ; il est identifié sous le nom INCI : Polyacrylate Crosspolymer-6 ;
SOLAGUM™ TARA est une gomme de Tara utilisée comme agent de texture et agent stabilisant d'émulsions identifié sous le nom INCI : Caesalpinia Spinosa Gum ;
Fluidifeel™Easy est un mélange de lauryl polyglucoside de degré de polymérisation de 1,20, de myristyl polyglucoside de degré de polymérisation de 1,20, et de polyglycéryl-6 laurate, utilisé comme agent émulsionnant de type huile-dans-eau pour préparer des émulsions cosmétiques.
Xylishine™ est un agent actif destiné améliorer la brillance des cheveux , identifié sous le nom INCI : Xylitylglucoside (and) Anhydroxylitol (and) Maltitol (and) Xylitol (and) Pelvetia Canaliculata Extract); LANOL™ 2681 est une phase grasse avec des propriétés émollientes utilisée pour la préparation de compositions cosmétiques identifiée sous le nom INCI : Coco-Caprylate/Caprate ;
SEA SATIN™ est un extrait huileux de la plante marine béta-maritinta, utilisé dans des compositions cosmétiques pour ses propriétés d'amélioration du volume, de la brillance, de la souplesse des cheveux ; il est identifié sous le nom INCI : Caprylic/Capric Triglycéride (and) Beta Vulgaris (Beet) Root Extract) ; Alariane™ est un ingrédient utilisé pour son effet protecteur des fibres des cheveux exposés aux stress extérieurs (pollution, rayons UV) ; il est identifié sous le nom INCI : Aqua (and) Butylene Glycol (and) Alaria Esculenta Extract.

## Revendications

**1.** Utilisation d'une composition (C) comprenant pour 100% de sa masse,

- de 10% massique à 90% massique d'au moins un polymère poly(farnésène) ayant une masse molaire moyenne en nombre supérieure ou égale 10.000 g/mol et inférieure ou égale à 120.000 g/mol, ledit polymère poly(farnésène) étant partiellement ou totalement hydrogéné et/ou fonctionnalisé avec des radicaux hydroxyle, et
- de 10% massique à 90% massique d'un mélange (M) d'hydrocarbures dans lequel au moins 94% massique des dits hydrocarbures comportent de quinze à dix-neuf atomes de carbone,

pour empêcher et/ou ralentir l'apparition d'effets inesthétiques sur les cheveux humains suite à l'exposition à une chaleur intense.

**2.** Utilisation selon la revendication 1, **caractérisée en ce que** ledit mélange (M) d'hydrocarbures compris dans ladite composition (C) est un mélange d'hydrocarbures saturés comprenant, pour 100% de sa masse :

- de 15% à 20% massique d'alcanes (linéaires, iso-alcanes et cyclo-alcanes) comportant 17 atomes de carbone,
- de 70% à 75% massique d'alcanes (linéaires, iso-alcanes et cyclo-alcanes) comportant 18 atomes de carbone, et
- de 4% à 6% massique d'alcanes (linéaires, iso-alcanes et cyclo-alcanes) comportant 19 atomes de carbone.

**3.** Utilisation selon la revendication 2, **caractérisée en ce que** ledit mélange (M) d'hydrocarbures présent dans ladite composition (C) est un mélange d'hydrocarbures saturés comprenant pour 100% de sa masse :

- 3,8% d'alcanes linéaires comportant de quinze à dix-neuf atomes de carbone,
- 96,2% d'iso-alcanes comportant de quinze à dix-neuf atomes de carbone, et
- moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

**4.** Utilisation selon l'une quelconque des revendications 1 à 3, **caractérisée en ce que** ledit polymère poly(farnésène) présent dans ladite composition (C), a une masse molaire moyenne en nombre supérieure ou égale 20.000 g/mol et inférieure ou égale à 90.000 g/mol, et en particulier une masse molaire moyenne en nombre supérieure ou égale 40.000 g/mol et inférieure ou égale à 80.000 g/mol.

**5.** Utilisation selon la revendication 4, **caractérisée en ce que** ledit polymère poly(farnésène) présent dans ladite composition (C), est totalement hydrogéné, fonctionnalisé avec des radicaux hydroxyle et a une masse molaire moyenne en nombre égale à 71.000 g/mole.

**6.** Utilisation selon l'une quelconque des revendications 1 à 5, **caractérisée en ce que** ladite composition (C) comprend pour 100% de sa masse,

- 40% massique d'un polymère poly(farnésène) totalement hydrogéné, fonctionnalisé avec des groupes hydroxyle et ayant une masse molaire moyenne en nombre égale à 71.000, et
- 60% massique d'un mélange (M) un mélange d'hydrocarbures saturés comprenant, pour 100% de sa masse :

  - 3,8% d'alcanes linéaires comportant de quinze à dix-neuf atomes de carbone,
  - 96,2% d'iso-alcanes comportant de quinze à dix-neuf atomes de carbone, et
  - moins de 0,1% de cycloalcanes comportant de quinze à dix-neuf atomes de carbone,

  et dans lequel :

  - 17,1% massique d'alcanes (linéaires, iso-alcanes et cyclo-alcanes) comportent 17 atomes de carbone,
  - 72,5% massique d'alcanes (linéaires, iso-alcanes et cyclo-alcanes) comportent 18 atomes de carbone, et
  - 4,7% massique d'alcanes (linéaires, iso-alcanes et cyclo-alcanes) comportent 19 atomes de carbone.

**7.** Utilisation selon l'une quelconque des revendications 1 à 6, **caractérisée en ce que** ladite composition (C) est mise en œuvre au sein d'une formulation cosmétique (F) en comprenant de 0,2% à 40% massique pour 100% de sa masse.

**8.** Utilisation selon la revendication 7, **caractérisée en ce que** ladite formulation (F) est une émulsion (E) d'une phase aqueuse (A) et d'une phase grasse (G) comprenant pour 100% de sa masse :

- de 50% à 98% massique, de ladite phase aqueuse (A), et
- de 2% à 50% massique, de ladite phase grasse (G),

ladite phase grasse (G) comprenant, pour 100% de sa masse :

- de 10% à 80% massique, de ladite composition (C),
- de 0,5% à 20% massique d'un agent tensioactif émulsionnant (S) ou d'un mélange d'agents tensioactifs émulsionnants ($M_S$) du type huile-dans-eau ou du type eau-dans huile, et
- de 0% à 89,5% massique, d'une huile ou de plusieurs huiles et/ou d'une cire, ladite cire étant différente de ladite composition (C).

**9.** Utilisation selon la revendication 7, **caractérisée en ce que** ladite composition (C) est mise en œuvre au sein d'une formulation cosmétique (F) en comprenant de 1% à 10% massique et plus particulièrement de 2% à 5% massique, pour 100% de sa masse.

**10.** Procédé de séchage des cheveux **caractérisé en ce qu'**il comprend les étapes successives suivantes:

- une étape a) d'application sur les cheveux d'une formulation cosmétique (F) comprenant pour 100% de sa masse de 0,2% à 40% massique d'une composition (C), ladite composition (C) comprenant pour 100% de sa masse,

  - de 10% massique à 90% massique d'au moins un polymère poly(farnésène) ayant une masse molaire moyenne en nombre supérieure ou égale 10.000 g/mol et inférieure ou égale à 120.000 g/mol, ledit polymère poly(farnésène) étant partiellement ou totalement hydrogéné et/ou fonctionnalisé avec des radicaux hydroxyle, et
  - de 10% massique à 90% massique d'un mélange (M) d'hydrocarbures dans lequel au moins 94% massique des dits hydrocarbures comportent de quinze à dix-neuf atomes de carbone,

- optionnellement, une étape b) au cours de laquelle à l'issue de l'étape a), les cheveux sont laissés au repos à température ambiante pendant une durée de 5 à 60 minutes puis
- une étape c) de séchage des cheveux à l'issue de l'étape a) ou optionnellement à l'issue de l'étape b), au moyen d'un appareil chauffant tel qu'un sèche-cheveux ou un casque chauffant.

**11.** Procédé selon la revendication 10, **caractérisé en ce que** ce que ledit mélange (M) d'hydrocarbures présent dans la composition (C) comprise dans la formulation (F) mise en œuvre à l'étape a), est un mélange d'hydrocarbures saturés comprenant, pour 100% de sa masse :

- de 15% à 20% massique d'alcanes (linéaires, iso-alcanes et cyclo-alcanes) comportant 17 atomes de carbone,
- de 70% à 75% massique d'alcanes (linéaires, iso-alcanes et cyclo-alcanes) comportant 18 atomes de carbone, et
- de 4% à 6% massique d'alcanes (linéaires, iso-alcanes et cyclo-alcanes) comportant 19 atomes de carbone.

**12.** Procédé selon la revendication 11, **caractérisé en ce que** ledit mélange (M) d'hydrocarbures présent dans la composition (C) comprise dans la formulation (F) mise en œuvre à l'étape a), est un mélange d'hydrocarbures saturés comprenant, pour 100% de sa masse, :

- 3,8% d'alcanes linéaires comportant de quinze à dix-neuf atomes de carbone,
- 96,2% d'iso-alcanes comportant de quinze à dix-neuf atomes de carbone, et
- moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

**13.** Procédé selon l'une des revendications 10 à 12, **caractérisé en ce que** ce que ledit polymère poly(farnésène) présent dans la composition (C) comprise dans la formulation (F) mise en œuvre à l'étape a), a une masse molaire moyenne en nombre supérieure ou égale 20.000 g/mol et inférieure ou égale à 90.000 g/mol, et en particulier une masse molaire moyenne en nombre supérieure ou égale 40.000 g/mol et inférieure ou égale à 80.000 g/mol.

**14.** Procédé selon la revendication 13, **caractérisé en ce que** ce que ledit polymère poly(farnésène) présent dans ladite composition (C) comprise dans la formulation (F) mise en œuvre à l'étape a), est totalement hydrogéné, fonctionnalisé avec des radicaux hydroxyle et a une masse molaire moyenne en nombre égale à 71.000 g/mole.

**15.** Procédé selon l'une des revendications 10 à 14, **caractérisé en ce que** ce que ladite composition (C) comprise dans la formulation (F) mise en œuvre à l'étape a), comprend pour 100% de sa masse,

- 40% massique d'un polymère poly(farnésène) totalement hydrogéné, fonctionnalisé avec des groupes hydroxyle et ayant une masse molaire moyenne en nombre égale à 71.000, et
- 60% massique d'un mélange (M) un mélange d'hydrocarbures saturés comprenant, pour 100% de sa masse :

  - 3,8% d'alcanes linéaires comportant de quinze à dix-neuf atomes de carbone,
  - 96,2% d'iso-alcanes comportant de quinze à dix-neuf atomes de carbone, et
  - moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

**16.** Procédé selon l'une quelconque des revendications 10 à 15, **caractérisé en ce que** ladite composition (C) est mise en œuvre au sein d'une émulsion (E) d'une phase aqueuse (A) et d'une phase grasse (G) comprenant pour 100% de sa masse :

- de 50% à 98% massique, de ladite phase aqueuse (A), et
- de 2% à 50% massique, de ladite phase grasse (G),

ladite phase grasse (G) comprenant, pour 100% de sa masse :

- de 10% à 80% massique, de ladite composition (C),
- de 0,5% à 20% massique d'un agent tensioactif émulsionnant (S) ou d'un mélange d'agents tensioactifs émulsionnants ($M_S$) du type huile-dans-eau ou du type eau-dans huile, et
- de 0% à 89,5% massique, d'une huile ou de plusieurs huiles et/ou d'une cire, ladite cire étant différente de ladite composition (C).

**17.** Procédé selon l'une quelconque des revendications 10 à 15, **caractérisé en ce que** ladite composition (C) est mise en œuvre au sein d'une formulation cosmétique (F) en comprenant pour 100% de sa masse, de 1% à 10% massique et plus particulièrement de 2% à 5% massique.

**18.** Procédé de mise en forme des cheveux **caractérisé en ce qu'**il comprend les étapes successives suivantes :

- une étape d) d'application sur les cheveux secs ou humides, d'une formulation cosmétique (F) comprenant pour 100% de sa masse 0,2% à 40% massique d'une composition (C) comprenant pour 100% de sa masse,

  - de 10% massique à 90% massique d'au moins un polymère poly(farnésène) ayant une masse molaire moyenne en nombre supérieure ou égale 10.000 g/mol et inférieure ou égale à 120.000 g/mol, ledit polymère poly(farnésène) étant partiellement ou totalement hydrogéné et/ou fonctionnalisé avec des radicaux hydroxyle, et
  - de 10% massique à 90% massique d'un mélange (M) d'hydrocarbures dans lequel au moins 94% massique des dits hydrocarbures comportent de quinze à dix-neuf atomes de carbone.

- optionnellement, une étape e) au cours de laquelle à l'issue de l'étape d), les cheveux sont laissés au repos à température ambiante pendant une durée de 5 à 60 minutes puis,
- une étape f) de mise en forme des cheveux à l'issue de l'étape d) ou optionnellement à l'issue de l'étape e), au moyen d'un appareil chauffant, tel qu'un fer à lisser ou un fer à boucler.

**19.** Procédé selon la revendication 18, **caractérisé en ce que** ce que ledit mélange (M) d'hydrocarbures présent dans la composition (C) comprise dans la formulation (F) mise en œuvre à l'étape d), est un mélange d'hydrocarbures saturés comprenant, pour 100% de sa masse :

- de 15% à 20% massique d'alcanes (linéaires, iso-alcanes et cyclo-alcanes) comportant 17 atomes de carbone,
- de 70% à 75% massique d'alcanes (linéaires, iso-alcanes et cyclo-alcanes) comportant 18 atomes de carbone, et
- de 4% à 6% massique d'alcanes (linéaires, iso-alcanes et cyclo-alcanes) comportant 19 atomes de carbone.

**20.** Procédé selon la revendication 19, **caractérisé en ce que** ledit mélange (M) d'hydrocarbures présent dans la composition (C) comprise dans la formulation (F) mise en œuvre à l'étape d), est un mélange d'hydrocarbures saturés comprenant, pour 100% de sa masse :

- 3,8% d'alcanes linéaires comportant de quinze à dix-neuf atomes de carbone,
- 96,2% d'iso-alcanes comportant de quinze à dix-neuf atomes de carbone, et
- moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

**21.** Procédé selon l'une des revendications 18 à 20, **caractérisé en ce que** ce que ledit polymère poly(farnésène) présent dans la composition (C) comprise dans la formulation (F) mise en œuvre à l'étape d), a une masse molaire moyenne en nombre supérieure ou égale 20.000 g/mol et inférieure ou égale à 90.000 g/mol, et en particulier une masse molaire moyenne en nombre supérieure ou égale 40.000 g/mol et inférieure ou égale à 80.000 g/mol.

**22.** Procédé selon la revendication 21, **caractérisé en ce que** ce que ledit polymère poly(farnésène) présent dans ladite composition (C) comprise dans la formulation (F) mise en œuvre à l'étape d), est totalement hydrogéné, fonctionnalisé avec des radicaux hydroxyle et a une masse molaire moyenne en nombre égale à 71.000 g/mole.

**23.** Procédé selon l'une des revendications 18 à 22, **caractérisé en ce que** ce que ladite composition (C) comprise dans la formulation (F) mise en œuvre à l'étape a), comprend pour 100% de sa masse,

- 40% massique d'un polymère poly(farnésène) totalement hydrogéné, fonctionnalisé avec des groupes hydroxyle et ayant une masse molaire moyenne en nombre égale à 71.000, et
- 60% massique d'un mélange (M) un mélange d'hydrocarbures saturés comprenant, pour 100% de sa masse :
  - 3,8% d'alcanes linéaires comportant de quinze à dix-neuf atomes de carbone,
  - 96,2% d'iso-alcanes comportant de quinze à dix-neuf atomes de carbone, et
  - moins de 0,1% de cycloalcanes comportant de quinze à dix-neuf atomes de carbone,

et dans lequel :

- 17,1% massique d'alcanes (linéaires, iso-alcanes et cyclo-alcanes) comportent 17 atomes de carbone,
- 72,5% massique d'alcanes (linéaires, iso-alcanes et cyclo-alcanes) comportent 18 atomes de carbone, et
- 4,7% massique d'alcanes (linéaires, iso-alcanes et cyclo-alcanes) comportent 19 atomes de carbone.

**24.** Procédé selon l'une quelconque des revendications 18 à 23, **caractérisé en ce que** ladite composition (C) est mise en œuvre au sein d'une émulsion (E) d'une phase aqueuse (A) et d'une phase grasse (G) comprenant pour 100% de sa masse :

- de 50% à 98% massique, de ladite phase aqueuse (A), et
- de 2% à 50% massique, de ladite phase grasse (G),

ladite phase grasse (G) comprenant, pour 100% de sa masse :

- de 10% à 80% massique, de ladite composition (C),
- de 0,5% à 20% massique d'un agent tensioactif émulsionnant (S) ou d'un mélange d'agents tensioactifs émulsionnants ($M_S$) du type huile-dans-eau ou du type eau-dans huile, et
- de 0% à 89,5% massique, d'une huile ou de plusieurs huiles et/ou d'une cire, ladite cire étant différente de ladite composition (C).

**25.** Procédé selon l'une quelconque des revendications 18 à 23, **caractérisé en ce que** ladite composition (C) est mise en œuvre au sein d'une formulation cosmétique (F) en comprenant pour 100% de sa masse, de 1% à 10% massique et plus particulièrement de 2% à 5% massique.

## Patentansprüche

**1.** Verwendung einer Zusammensetzung (C), umfassend pro 100 % ihrer Masse,

- zu 10 Masse-% bis 90 Masse-% mindestens ein Poly(farnesen)-Polymer, das eine zahlenmittlere Molmasse von mehr als oder gleich 10.000 g/mol und weniger als oder gleich 120.000 g/mol besitzt, wobei dieses

Poly(farnesen)-Polymer teilweise oder vollständig hydriert und/oder mit Hydroxylradikalen funktionalisiert ist, und

- zu 10 Masse-% bis 90 Masse-% ein Gemisch (M) von Kohlenwasserstoffen, wobei mindestens 94 Masse-% der Kohlenwasserstoffe fünfzehn bis neunzehn Kohlenstoffatome aufweisen,

zum Verhindern und/oder Verlangsamen des Auftretens unansehnlicher Auswirkungen an menschlichem Haar nach der Aussetzung gegenüber intensiver Hitze.

2. Verwendung nach Anspruch 1, **dadurch gekennzeichnet, dass** das Gemisch (M) von Kohlenwasserstoffen, das in der Zusammensetzung (C) enthalten ist, ein Gemisch von gesättigten Kohlenwasserstoffen ist, umfassend pro 100 % seiner Masse:

   - zu 15 bis 20 Masse-% Alkane (lineare, Isoalkane und Cycloalkane), die 17 Kohlenstoffatome aufweisen,
   - zu 70 bis 75 Masse-% Alkane (lineare, Isoalkane und Cycloalkane), die 18 Kohlenstoffatome aufweisen, und
   - zu 4 bis 6 Masse-% Alkane (lineare, Isoalkane und Cycloalkane), die 19 Kohlenstoffatome aufweisen.

3. Verwendung nach Anspruch 2, **dadurch gekennzeichnet, dass** das Gemisch (M) von Kohlenwasserstoffen, das in der Zusammensetzung (C) vorliegt, ein Gemisch von gesättigten Kohlenwasserstoffen ist, umfassend pro 100 % seiner Masse:

   - zu 3,8 % lineare Alkane, die fünfzehn bis neunzehn Kohlenstoffatome aufweisen,
   - zu 96,2 % Isoalkane, die fünfzehn bis neunzehn Kohlenstoffatome aufweisen, und
   - zu weniger als 0,1 % Cycloalkane, die fünfzehn bis neunzehn Kohlenstoffatome aufweisen,

   und wobei:

     - 17,1 Masse-% der Alkane (lineare, Isoalkane und Cycloalkane) 17 Kohlenstoffatome aufweisen,
     - 72,5 Masse-% der Alkane (lineare, Isoalkane und Cycloalkane) 18 Kohlenstoffatome aufweisen, und
     - 4,7 Masse-% der Alkane (lineare, Isoalkane und Cycloalkane) 19 Kohlenstoffatome aufweisen.

4. Verwendung nach einem der Ansprüche 1 bis 3,
**dadurch gekennzeichnet, dass** das Poly(farnesen)-Polymer, das in der Zusammensetzung (C) vorliegt, eine zahlenmittlere Molmasse von mehr als oder gleich 20.000 g/mol und weniger als oder gleich 90.000 g/mol, insbesondere eine zahlenmittlere Molmasse von mehr als oder gleich 40.000 g/mol und weniger als oder gleich 80.000 g/mol besitzt.

5. Verwendung nach Anspruch 4, **dadurch gekennzeichnet, dass** das Poly(farnesen)-Polymer das in der Zusammensetzung (C) vorliegt, vollständig hydriert ist, mit Hydroxylradikalen funktionalisiert ist und eine zahlenmittlere Molmasse von 71.000 g/mol besitzt.

6. Verwendung nach einem der Ansprüche 1 bis 5,
**dadurch gekennzeichnet, dass** die Zusammensetzung (C) pro 100 % ihrer Masse umfasst,

   - zu 40 Masse-% ein vollständig hydriertes Poly(farnesen)-Polymer, das mit Hydroxylgruppen funktionalisiert ist und eine zahlenmittlere Molmasse von 71.000 besitzt, und
   - zu 60 Masse-% ein Gemisch (M) aus gesättigten Kohlenwasserstoffen, umfassend pro 100 % seiner Masse:
   - zu 3,8 % lineare Alkane, die fünfzehn bis neunzehn Kohlenstoffatome aufweisen,
   - zu 96,2 % Isoalkane, die fünfzehn bis neunzehn Kohlenstoffatome aufweisen, und
   - zu weniger als 0,1 % Cycloalkane, die fünfzehn bis neunzehn Kohlenstoffatome aufweisen,

   und wobei:

     - 17,1 Masse-% der Alkane (lineare, Isoalkane und Cycloalkane) 17 Kohlenstoffatome aufweisen,
     - 72,5 Masse-% der Alkane (lineare, Isoalkane und Cycloalkane) 18 Kohlenstoffatome aufweisen, und
     - 4,7 Masse-% der Alkane (lineare, Isoalkane und Cycloalkane) 19 Kohlenstoffatome aufweisen.

7. Verwendung nach einem der Ansprüche 1 bis 6,
**dadurch gekennzeichnet, dass** die Zusammensetzung (C) in einer kosmetischen Formulierung (F) eingesetzt wird, umfassend 0,2 bis 40 Masse-% pro 100 % ihrer Masse.

8. Verwendung nach Anspruch 7, **dadurch gekennzeichnet, dass** die Formulierung (F) eine Emulsion (E) aus einer

wässrigen Phase (A) und einer Fettphase (G) ist, umfassend pro 100 % ihrer Masse:

- zu 50 bis 98 Masse-% die wässrige Phase (A), und
- zu 2 bis 50 Masse-% die Fettphase (G),

die Fettphase (G) umfassend pro 100 % ihrer Masse:

  - zu 10 bis 80 Masse-% die Zusammensetzung (C),
  - zu 0,5 bis 20 Masse-% ein emulgierendes Tensid (S) oder ein Gemisch von emulgierenden Tensiden ($M_S$) von der Art Öl-in-Wasser oder Wasser-in-Öl, und
  - zu 0 bis 89,5 Masse-% ein Öl oder mehrere Öle und/oder ein Wachs, wobei sich das Wachs von der Zusammensetzung (C) unterscheidet.

**9.** Verwendung nach Anspruch 7, **dadurch gekennzeichnet, dass** die Zusammensetzung (C) in einer kosmetischen Formulierung (F) eingesetzt wird, umfassend 1 bis 10 Masse-% und insbesondere 2 bis 5 Masse-% pro 100 % ihrer Masse.

**10.** Haartrocknungsverfahren, **dadurch gekennzeichnet, dass** es die folgenden aufeinanderfolgenden Schritte umfasst:

- einen Schritt a) zum Anwenden einer kosmetischen Formulierung (F) auf das Haar, umfassend pro 100 % ihrer Masse zu 0,2 bis 40 % eine Zusammensetzung (C), die Zusammensetzung (C) umfassend pro 100 % ihrer Masse,
- zu 10 Masse-% bis 90 Masse-% mindestens ein Poly(farnesen)-Polymer, das eine zahlenmittlere Molmasse von mehr als oder gleich 10.000 g/mol und weniger als oder gleich 120.000 g/mol besitzt, wobei dieses Poly(farnesen)-Polymer teilweise oder vollständig hydriert und/oder mit Hydroxylradikalen funktionalisiert ist, und
- zu 10 Masse-% bis 90 Masse-% ein Gemisch (M) von Kohlenwasserstoffen, wobei mindestens 94 Masse-% der Kohlenwasserstoffe fünfzehn bis neunzehn Kohlenstoffatome aufweisen,
- optional einen Schritt b), während dessen nach Schritt a) das Haar während einer Dauer von 5 bis 60 Minuten bei Raumtemperatur ruhen gelassen wird, dann
- einen Schritt c) zum Trocknen des Haars nach Schritt a) oder optional nach Schritt b) mittels einer Erhitzungsvorrichtung wie eines Föhns oder einer erhitzenden Haube.

**11.** Verfahren nach Anspruch 10, **dadurch gekennzeichnet, dass** das Gemisch (M) von Kohlenwasserstoffen, das in der Zusammensetzung (C) vorliegt, die in der Formulierung (F) enthalten ist, die in Schritt a) eingesetzt wird, ein Gemisch von gesättigten Kohlenwasserstoffen ist, umfassend pro 100 % seiner Masse:

- zu 15 bis 20 Masse-% Alkane (lineare, Isoalkane und Cycloalkane), die 17 Kohlenstoffatome aufweisen,
- zu 70 bis 75 Masse-% Alkane (lineare, Isoalkane und Cycloalkane), die 18 Kohlenstoffatome aufweisen, und
- zu 4 bis 6 Masse-% Alkane (lineare, Isoalkane und Cycloalkane), die 19 Kohlenstoffatome aufweisen.

**12.** Verfahren nach Anspruch 11, **dadurch gekennzeichnet, dass** das Gemisch (M) von Kohlenwasserstoffen, das in der Zusammensetzung (C) vorliegt, die in der Formulierung (F) enthalten ist, die in Schritt a) eingesetzt wird, ein Gemisch von gesättigten Kohlenwasserstoffen ist, umfassend pro 100 % seiner Masse,:

- zu 3,8 % lineare Alkane, die fünfzehn bis neunzehn Kohlenstoffatome aufweisen,
- zu 96,2 % Isoalkane, die fünfzehn bis neunzehn Kohlenstoffatome aufweisen, und
- zu weniger als 0,1 % Cycloalkane, die fünfzehn bis neunzehn Kohlenstoffatome aufweisen,

und wobei:

  - 17,1 Masse-% der Alkane (lineare, Isoalkane und Cycloalkane) 17 Kohlenstoffatome aufweisen,
  - 72,5 Masse-% der Alkane (lineare, Isoalkane und Cycloalkane) 18 Kohlenstoffatome aufweisen, und
  - 4,7 Masse-% der Alkane (lineare, Isoalkane und Cycloalkane) 19 Kohlenstoffatome aufweisen.

**13.** Verfahren nach einem der Ansprüche 10 bis 12,
**dadurch gekennzeichnet, dass** das Poly(farnesen)-Polymer, das in der Zusammensetzung (C) vorliegt, die in der Formulierung (F) enthalten ist, die in Schritt a) eingesetzt wird, eine zahlenmittlere Molmasse von mehr als oder gleich 20.000 g/mol und weniger als oder gleich 90.000 g/mol, und insbesondere eine zahlenmittlere Molmasse von mehr

als oder gleich 40.000 g/mol und weniger als oder gleich 80.000 g/mol besitzt.

**14.** Verfahren nach Anspruch 13, **dadurch gekennzeichnet, dass** das Poly(farnesen)-Polymer, das in der Zusammensetzung (C) vorliegt, die in der Formulierung (F) enthalten ist, die in Schritt a) eingesetzt wird, vollständig hydriert ist, mit Hydroxylradikalen funktionalisiert ist und eine zahlenmittlere Molmasse von 71.000 g/mol besitzt.

**15.** Verfahren nach einem der Ansprüche 10 bis 14,
**dadurch gekennzeichnet, dass** die Zusammensetzung (C), die in der Formulierung (F) enthalten ist, die in Schritt a) eingesetzt wird, pro 100 % ihrer Masse umfasst,

- zu 40 Masse-% ein vollständig hydriertes Poly(farnesen)-Polymer, das mit Hydroxylgruppen funktionalisiert ist und eine zahlenmittlere Molmasse von 71.000 besitzt, und
- zu 60 Masse-% ein Gemisch (M) aus gesättigten Kohlenwasserstoffen, umfassend pro 100 % seiner Masse:

    - zu 3,8 % lineare Alkane, die fünfzehn bis neunzehn Kohlenstoffatome aufweisen,
    - zu 96,2 % Isoalkane, die fünfzehn bis neunzehn Kohlenstoffatome aufweisen, und
    - zu weniger als 0,1 % Cycloalkane, die fünfzehn bis neunzehn Kohlenstoffatome aufweisen,

    und wobei:

        - 17,1 Masse-% der Alkane (lineare, Isoalkane und Cycloalkane) 17 Kohlenstoffatome aufweisen,
        - 72,5 Masse-% der Alkane (lineare, Isoalkane und Cycloalkane) 18 Kohlenstoffatome aufweisen, und
        - 4,7 Masse-% der Alkane (lineare, Isoalkane und Cycloalkane) 19 Kohlenstoffatome aufweisen.

**16.** Verfahren nach einem der Ansprüche 10 bis 15,
**dadurch gekennzeichnet, dass** die Zusammensetzung (C) in einer Emulsion (E) aus einer wässrigen Phase (A) und einer Fettphase (G) eingesetzt wird, umfassend pro 100 % ihrer Masse:

- zu 50 bis 98 Masse-% die wässrige Phase (A), und
- zu 2 bis 50 Masse-% die Fettphase (G),

die Fettphase (G) umfassend pro 100 % ihrer Masse:

    - zu 10 bis 80 Masse-% die Zusammensetzung (C),
    - zu 0,5 bis 20 Masse-% ein emulgierendes Tensid (S) oder ein Gemisch von emulgierenden Tensiden ($M_S$) von der Art Öl-in-Wasser oder Wasser-in-Öl, und
    - zu 0 bis 89,5 Masse-% ein Öl oder mehrere Öle und/oder ein Wachs, wobei sich das Wachs von der Zusammensetzung (C) unterscheidet.

**17.** Verfahren nach einem der Ansprüche 10 bis 15,
**dadurch gekennzeichnet, dass** die Zusammensetzung (C) in einer kosmetischen Formulierung (F) eingesetzt wird, umfassend pro 100 % ihrer Masse 1 bis 10 Masse-% und insbesondere 2 bis 5 Masse-%.

**18.** Haarformgebungsverfahren, **dadurch gekennzeichnet, dass** es die folgenden aufeinanderfolgenden Schritte umfasst:

- einen Schritt a) zum Anwenden einer kosmetischen Formulierung (F) auf trockenes oder feuchtes Haar, umfassend pro 100 % ihrer Masse zu 0,2 bis 40 % eine Zusammensetzung (C), umfassend pro 100 % ihrer Masse,
- zu 10 Masse-% bis 90 Masse-% mindestens ein Poly(farnesen)-Polymer, das eine zahlenmittlere Molmasse von mehr als oder gleich 10.000 g/mol und weniger als oder gleich 120.000 g/mol besitzt, wobei dieses Poly(farnesen)-Polymer teilweise oder vollständig hydriert und/oder mit Hydroxylradikalen funktionalisiert ist, und
- zu 10 Masse-% bis 90 Masse-% ein Gemisch (M) von Kohlenwasserstoffen, wobei mindestens 94 Masse-% der Kohlenwasserstoffe fünfzehn bis neunzehn Kohlenstoffatome aufweisen,
- optional einen Schritt e), während dessen nach Schritt d) das Haar während einer Dauer von 5 bis 60 Minuten bei Raumtemperatur ruhen gelassen wird, dann,
- einen Schritt f) für die Haarformgebung nach Schritt d) oder optional nach Schritt e), mittels einer Erhitzungsvorrichtung, wie eines Glätteisens oder eines Lockenstabs.

**19.** Verfahren nach Anspruch 18, **dadurch gekennzeichnet, dass** das Gemisch (M) von Kohlenwasserstoffen, das in der Zusammensetzung (C) vorliegt, die in der Formulierung (F) enthalten ist, die in Schritt d) eingesetzt wird, ein Gemisch von gesättigten Kohlenwasserstoffen ist, umfassend pro 100 % seiner Masse:

- zu 15 bis 20 Masse-% Alkane (lineare, Isoalkane und Cycloalkane), die 17 Kohlenstoffatome aufweisen,
- zu 70 bis 75 Masse-% Alkane (lineare, Isoalkane und Cycloalkane), die 18 Kohlenstoffatome aufweisen, und
- zu 4 bis 6 Masse-% Alkane (lineare, Isoalkane und Cycloalkane), die 19 Kohlenstoffatome aufweisen.

**20.** Verfahren nach Anspruch 19, **dadurch gekennzeichnet, dass** das Gemisch (M) von Kohlenwasserstoffen, das in der Zusammensetzung (C) vorliegt, die in der Formulierung (F) enthalten ist, die in Schritt d) eingesetzt wird, ein Gemisch von gesättigten Kohlenwasserstoffen ist, umfassend pro 100 % seiner Masse,:

- zu 3,8 % lineare Alkane, die fünfzehn bis neunzehn Kohlenstoffatome aufweisen,
- zu 96,2 % Isoalkane, die fünfzehn bis neunzehn Kohlenstoffatome aufweisen, und
- zu weniger als 0,1 % Cycloalkane, die fünfzehn bis neunzehn Kohlenstoffatome aufweisen,

und wobei:

  - 17,1 Masse-% der Alkane (lineare, Isoalkane und Cycloalkane) 17 Kohlenstoffatome aufweisen,
  - 72,5 Masse-% der Alkane (lineare, Isoalkane und Cycloalkane) 18 Kohlenstoffatome aufweisen, und
  - 4,7 Masse-% der Alkane (lineare, Isoalkane und Cycloalkane) 19 Kohlenstoffatome aufweisen.

**21.** Verfahren nach einem der Ansprüche 18 bis 20,
**dadurch gekennzeichnet, dass** das Poly(farnesen)-Polymer, das in der Zusammensetzung (C) vorliegt, die in der Formulierung (F) enthalten ist, die in Schritt d) eingesetzt wird, eine zahlenmittlere Molmasse von mehr als oder gleich 20.000 g/mol und weniger als oder gleich 90.000 g/mol, und insbesondere eine zahlenmittlere Molmasse von mehr als oder gleich 40.000 g/mol und weniger als oder gleich 80.000 g/mol besitzt.

**22.** Verfahren nach Anspruch 21, **dadurch gekennzeichnet, dass** das Poly(farnesen)-Polymer, das in der Zusammensetzung (C) vorliegt, die in der Formulierung (F) enthalten ist, die in Schritt d) eingesetzt wird, vollständig hydriert ist, mit Hydroxylradikalen funktionalisiert ist und eine zahlenmittlere Molmasse von 71.000 g/mol besitzt.

**23.** Verfahren nach einem der Ansprüche 18 bis 22,
**dadurch gekennzeichnet, dass** die Zusammensetzung (C), die in der Formulierung (F) enthalten ist, die in Schritt a) eingesetzt wird, pro 100 % ihrer Masse umfasst,

- zu 40 Masse-% ein vollständig hydriertes Poly(farnesen)-Polymer, das mit Hydroxylgruppen funktionalisiert ist und eine zahlenmittlere Molmasse von 71.000 besitzt, und
- zu 60 Masse-% ein Gemisch (M) aus gesättigten Kohlenwasserstoffen, umfassend pro 100 % seiner Masse:

  - zu 3,8 % lineare Alkane, die fünfzehn bis neunzehn Kohlenstoffatome aufweisen,
  - zu 96,2 % Isoalkane, die fünfzehn bis neunzehn Kohlenstoffatome aufweisen, und
  - zu weniger als 0,1 % Cycloalkane, die fünfzehn bis neunzehn Kohlenstoffatome aufweisen, und wobei:

    - 17,1 Masse-% der Alkane (lineare, Isoalkane und Cycloalkane) 17 Kohlenstoffatome aufweisen,
    - 72,5 Masse-% der Alkane (lineare, Isoalkane und Cycloalkane) 18 Kohlenstoffatome aufweisen, und
    - 4,7 Masse-% der Alkane (lineare, Isoalkane und Cycloalkane) 19 Kohlenstoffatome aufweisen.

**24.** Verfahren nach einem der Ansprüche 18 bis 23,
**dadurch gekennzeichnet, dass** die Zusammensetzung (C) in einer Emulsion (E) aus einer wässrigen Phase (A) und einer Fettphase (G) eingesetzt wird, umfassend pro 100 % ihrer Masse:

- zu 50 bis 98 Masse-% die wässrige Phase (A), und
- zu 2 bis 50 Masse-% die Fettphase (G),

die Fettphase (G) umfassend pro 100 % ihrer Masse:

  - zu 10 bis 80 Masse-% die Zusammensetzung (C),
  - zu 0,5 bis 20 Masse-% ein emulgierendes Tensid (S) oder ein Gemisch von emulgierenden Tensiden ($M_S$) von der Art Öl-in-Wasser oder Wasser-in-Öl, und

- zu 0 bis 89,5 Masse-% ein Öl oder mehrere Öle und/oder ein Wachs, wobei sich das Wachs von der Zusammensetzung (C) unterscheidet.

**25.** Verfahren nach einem der Ansprüche 18 bis 23,
**dadurch gekennzeichnet, dass** die Zusammensetzung (C) in einer kosmetischen Formulierung (F) eingesetzt wird, umfassend für 100 % ihrer Masse 1 bis 10 Masse-% und insbesondere 2 bis 5 Masse-%.

**Claims**

**1.** Use of a composition (C) comprising, for 100% of the mass thereof,

- from 10% by mass to 90% by mass of at least one poly(farnesene) polymer having a number-average molecular mass of greater than or equal to 10,000 g/mol and less than or equal to 120,000 g/mol, said poly(farnesene) polymer being partially or totally hydrogenated and/or functionalized with hydroxyl radicals, and
- from 10% by mass to 90% by mass of a mixture (M) of hydrocarbons in which at least 94% by mass of said hydrocarbons comprise from fifteen to nineteen carbon atoms,

for preventing and/or slowing the appearance of unattractive effects on human hair following exposure to intense heat.

**2.** Use according to claim 1, **characterized in that** said mixture (M) of hydrocarbons contained in said composition (C) is a mixture of saturated hydrocarbons comprising, for 100% of the mass thereof:

- from 15% to 20% by mass of alkanes (linear, isoalkanes and cycloalkanes) comprising 17 carbon atoms,
- from 70% to 75% by mass of alkanes (linear, isoalkanes and cycloalkanes) comprising 18 carbon atoms, and
- from 4% to 6% by mass of alkanes (linear, isoalkanes and cycloalkanes) comprising 19 carbon atoms.

**3.** Use according to claim 2, **characterized in that** said mixture (M) of hydrocarbons present in said composition (C) is a mixture of saturated hydrocarbons comprising, for 100% of the mass thereof:

- 3.8% linear alkanes comprising from fifteen to nineteen carbon atoms,
- 96.2% isoalkanes comprising from fifteen to nineteen carbon atoms, and
- less than 0.1% cycloalkanes comprising from fifteen to nineteen carbon atoms,

and wherein:

  - 17.1% by mass of alkanes (linear, isoalkanes and cycloalkanes) comprise 17 carbon atoms,
  - 72.5% by mass of alkanes (linear, isoalkanes and cycloalkanes) comprise 18 carbon atoms, and
  - 4.7% by mass of alkanes (linear, isoalkanes and cycloalkanes) comprise 19 carbon atoms.

**4.** Use according to any one of claims 1 to 3, **characterized in that** said poly(farnesene) polymer present in said composition (C) has a number-average molecular mass of greater than or equal to 20,000 g/mol and less than or equal to 90,000 g/mol, and in particular a number-average molecular mass of greater than or equal to 40,000 g/mol and less than or equal to 80,000 g/mol.

**5.** Use according to claim 4, **characterized in that** said poly(farnesene) polymer present in said composition (C) is totally hydrogenated, functionalized with hydroxyl radicals and has a number-average molecular mass equal to 71,000 g/mol.

**6.** Use according to any one of claims 1 to 5, **characterized in that** said composition (C) comprises, for 100% of the mass thereof,

- 40% by mass of a totally hydrogenated poly(farnesene) polymer functionalized with hydroxyl groups and having a number-average molecular mass equal to 71,000, and
- 60% by mass of a mixture (M) of saturated hydrocarbons comprising, for 100% of the mass thereof:

  - 3.8% linear alkanes comprising from fifteen to nineteen carbon atoms,
  - 96.2% isoalkanes comprising from fifteen to nineteen carbon atoms, and
  - less than 0.1% cycloalkanes comprising from fifteen to nineteen carbon atoms,

and wherein:

- 17.1% by mass of alkanes (linear, isoalkanes and cycloalkanes) comprise 17 carbon atoms,
- 72.5% by mass of alkanes (linear, isoalkanes and cycloalkanes) comprise 18 carbon atoms, and
- 4.7% by mass of alkanes (linear, isoalkanes and cycloalkanes) comprise 19 carbon atoms.

**7.** Use according to any one of claims 1 to 6, **characterized in that** said composition (C) is used in a cosmetic formulation (F) comprising from 0.2% to 40% by mass, for 100% of the mass thereof.

**8.** Use according to claim 7, **characterized in that** said formulation (F) is an emulsion (E) of an aqueous phase (A) and a fatty phase (G) comprising, for 100% of the mass thereof:

- from 50% to 98% by mass of said aqueous phase (A), and
- from 2% to 50% by mass of said fatty phase (G),

said fatty phase (G) comprising, for 100% of the mass thereof:

- from 10% to 80% by mass of said composition (C),
- from 0.5% to 20% by mass of an emulsifying surfactant (S) or a mixture of emulsifying surfactants ($M_S$) of the oil-in-water or water-in-oil type, and
- from 0% to 89.5% by mass of an oil or a plurality of oils and/or a wax, said wax being different from said composition (C).

**9.** Use according to claim 7, **characterized in that** said composition (C) is used in a cosmetic formulation (F) comprising from 1% to 10% by mass and more particularly from 2% to 5% by mass, for 100% of the mass thereof.

**10.** Method for drying the hair, **characterized in that** it comprises the following consecutive steps:

- a step a) of applying to the hair a cosmetic formulation (F) comprising, for 100% of the mass thereof, from 0.2% to 40% by mass of a composition (C), said composition (C) comprising, for 100% of the mass thereof,
- from 10% by mass to 90% by mass of at least one poly(farnesene) polymer having a number-average molecular mass of greater than or equal to 10,000 g/mol and less than or equal to 120,000 g/mol, said poly(farnesene) polymer being partially or totally hydrogenated and/or functionalized with hydroxyl radicals, and
- from 10% by mass to 90% by mass of a mixture (M) of hydrocarbons in which at least 94% by mass of said hydrocarbons comprise from fifteen to nineteen carbon atoms,
- optionally a step b) during which, at the end of step a), the hair is left to rest at room temperature for a duration of 5 to 60 minutes, then
- a step c) of drying the hair at the end of step a) or optionally at the end of step b), by means of a heating appliance such as a hair dryer or heated hood.

**11.** Method according to claim 10, **characterized in that** said mixture (M) of hydrocarbons present in the composition (C) contained in the formulation (F) used in step a) is a mixture of saturated hydrocarbons comprising, for 100% of the mass thereof:

- from 15% to 20% by mass of alkanes (linear, isoalkanes and cycloalkanes) comprising 17 carbon atoms,
- from 70% to 75% by mass of alkanes (linear, isoalkanes and cycloalkanes) comprising 18 carbon atoms, and
- from 4% to 6% by mass of alkanes (linear, isoalkanes and cycloalkanes) comprising 19 carbon atoms.

**12.** Method according to claim 11, **characterized in that** said mixture (M) of hydrocarbons present in the composition (C) contained in the formulation (F) used in step a) is a mixture of saturated hydrocarbons comprising, for 100% of the mass thereof:

- 3.8% linear alkanes comprising from fifteen to nineteen carbon atoms,
- 96.2% isoalkanes comprising from fifteen to nineteen carbon atoms, and
- less than 0.1% cycloalkanes comprising from fifteen to nineteen carbon atoms,

and wherein:

- 17.1% by mass of alkanes (linear, isoalkanes and cycloalkanes) comprise 17 carbon atoms,
- 72.5% by mass of alkanes (linear, isoalkanes and cycloalkanes) comprise 18 carbon atoms, and

- 4.7% by mass of alkanes (linear, isoalkanes and cycloalkanes) comprise 19 carbon atoms.

**13.** Method according to any of claims 10 to 12, **characterized in that** said poly(farnesene) polymer present in the composition (C) contained in the formulation (F) used in step a) has a number-average molecular mass of greater than or equal to 20,000 g/mol and less than or equal to 90,000 g/mol, and in particular a number-average molecular mass of greater than or equal to 40,000 g/mol and less than or equal to 80,000 g/mol.

**14.** Method according to claim 13, **characterized in that** said poly(farnesene) polymer present in said composition (C) contained in the formulation (F) used in step a) is totally hydrogenated, functionalized with hydroxyl radicals and has a number-average molecular mass equal to 71,000 g/mol.

**15.** Method according to any of claims 10 to 14, **characterized in that** said composition (C) contained in the formulation (F) used in step a) comprises, for 100% of the mass thereof,

- 40% by mass of a totally hydrogenated poly(farnesene) polymer functionalized with hydroxyl groups and having a number-average molecular mass equal to 71,000, and
- 60% by mass of a mixture (M) of saturated hydrocarbons comprising, for 100% of the mass thereof:

  - 3.8% linear alkanes comprising from fifteen to nineteen carbon atoms,
  - 96.2% isoalkanes comprising from fifteen to nineteen carbon atoms, and
  - less than 0.1% cycloalkanes comprising from fifteen to nineteen carbon atoms,

  and wherein:

    - 17.1% by mass of alkanes (linear, isoalkanes and cycloalkanes) comprise 17 carbon atoms,
    - 72.5% by mass of alkanes (linear, isoalkanes and cycloalkanes) comprise 18 carbon atoms, and
    - 4.7% by mass of alkanes (linear, isoalkanes and cycloalkanes) comprise 19 carbon atoms.

**16.** Method according to any one of claims 10 to 15, **characterized in that** said composition (C) is used in an emulsion (E) of an aqueous phase (A) and a fatty phase (G) comprising, for 100% of the mass thereof:

- from 50% to 98% by mass of said aqueous phase (A), and
- from 2% to 50% by mass of said fatty phase (G),

said fatty phase (G) comprising, for 100% of the mass thereof:

  - from 10% to 80% by mass of said composition (C),
  - from 0.5% to 20% by mass of an emulsifying surfactant (S) or a mixture of emulsifying surfactants ($M_S$) of the oil-in-water or water-in-oil type, and
  - from 0% to 89.5% by mass of an oil or a plurality of oils and/or a wax, said wax being different from said composition (C).

**17.** Method according to any one of claims 10 to 15, **characterized in that** said composition (C) is used in a cosmetic formulation (F) comprising, for 100% of the mass thereof, from 1% to 10% by mass, and more particularly from 2% to 5% by mass.

**18.** Method for shaping the hair, **characterized in that** it comprises the following consecutive steps:

- a step d) of applying, to dry or wet hair, a cosmetic formulation (F) comprising, for 100% of the mass thereof, 0.2% to 40% by mass of a composition (C) comprising, for 100% of the mass thereof,
- from 10% by mass to 90% by mass of at least one poly(farnesene) polymer having a number-average molecular mass of greater than or equal to 10,000 g/mol and less than or equal to 120,000 g/mol, said poly(farnesene) polymer being partially or totally hydrogenated and/or functionalized with hydroxyl radicals, and
- from 10% by mass to 90% by mass of a mixture (M) of hydrocarbons in which at least 94% by mass of said hydrocarbons comprise from fifteen to nineteen carbon atoms.
- optionally a step e) during which, at the end of step d), the hair is left to rest at room temperature for a duration of 5 to 60 minutes, then,
- a step f) of shaping the hair at the end of step d) or optionally at the end of step e), by means of a heating appliance such as a straightening iron or curling tongs.

**19.** Method according to claim 18, **characterized in that** said mixture (M) of hydrocarbons present in the composition (C) contained in the formulation (F) used in step d) is a mixture of saturated hydrocarbons comprising, for 100% of the mass thereof:

- from 15% to 20% by mass of alkanes (linear, isoalkanes and cycloalkanes) comprising 17 carbon atoms,
- from 70% to 75% by mass of alkanes (linear, isoalkanes and cycloalkanes) comprising 18 carbon atoms, and
- from 4% to 6% by mass of alkanes (linear, isoalkanes and cycloalkanes) comprising 19 carbon atoms.

**20.** Method according to claim 19, **characterized in that** said mixture (M) of hydrocarbons present in the composition (C) contained in the formulation (F) used in step d) is a mixture of saturated hydrocarbons comprising, for 100% of the mass thereof:

- 3.8% linear alkanes comprising from fifteen to nineteen carbon atoms,
- 96.2% isoalkanes comprising from fifteen to nineteen carbon atoms, and
- less than 0.1% cycloalkanes comprising from fifteen to nineteen carbon atoms,

and wherein:

  - 17.1% by mass of alkanes (linear, isoalkanes and cycloalkanes) comprise 17 carbon atoms,
  - 72.5% by mass of alkanes (linear, isoalkanes and cycloalkanes) comprise 18 carbon atoms, and
  - 4.7% by mass of alkanes (linear, isoalkanes and cycloalkanes) comprise 19 carbon atoms.

**21.** Method according to any of claims 18 to 20, **characterized in that** said poly(farnesene) polymer present in the composition (C) contained in the formulation (F) used in step d) has a number-average molecular mass of greater than or equal to 20,000 g/mol and less than or equal to 90,000 g/mol, and in particular a number-average molecular mass of greater than or equal to 40,000 g/mol and less than or equal to 80,000 g/mol.

**22.** Method according to claim 21, **characterized in that** said poly(farnesene) polymer present in said composition (C) contained in the formulation (F) used in step d) is totally hydrogenated, functionalized with hydroxyl radicals and has a number-average molecular mass equal to 71,000 g/mol.

**23.** Method according to any of claims 18 to 22, **characterized in that** said composition (C) contained in the formulation (F) used in step a) comprises, for 100% of the mass thereof,

- 40% by mass of a totally hydrogenated poly(farnesene) polymer functionalized with hydroxyl groups and having a number-average molecular mass equal to 71,000, and
- 60% by mass of a mixture (M) of saturated hydrocarbons comprising, for 100% of the mass thereof:
  - 3.8% linear alkanes comprising from fifteen to nineteen carbon atoms,
  - 96.2% isoalkanes comprising from fifteen to nineteen carbon atoms, and
  - less than 0.1% cycloalkanes comprising from fifteen to nineteen carbon atoms, and wherein:
    - 17.1% by mass of alkanes (linear, isoalkanes and cycloalkanes) comprise 17 carbon atoms,
    - 72.5% by mass of alkanes (linear, isoalkanes and cycloalkanes) comprise 18 carbon atoms, and
    - 4.7% by mass of alkanes (linear, isoalkanes and cycloalkanes) comprise 19 carbon atoms.

**24.** Method according to any one of claims 18 to 23, **characterized in that** said composition (C) is used in an emulsion (E) of an aqueous phase (A) and a fatty phase (G) comprising, for 100% of the mass thereof:

- from 50% to 98% by mass of said aqueous phase (A), and
- from 2% to 50% by mass of said fatty phase (G),

said fatty phase (G) comprising, for 100% of the mass thereof:

  - from 10% to 80% by mass of said composition (C),
  - from 0.5% to 20% by mass of an emulsifying surfactant (S) or a mixture of emulsifying surfactants ($M_S$) of the oil-in-water or water-in-oil type, and
  - from 0% to 89.5% by mass of an oil or a plurality of oils and/or a wax, said wax being different from said composition (C).

**25.** Method according to any one of claims 18 to 23, **characterized in that** said composition (C) is used in a cosmetic formulation (F) comprising, for 100% of the mass thereof, from 1% to 10% by mass, and more particularly from 2% to 5% by mass.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 201495210 A1
- FR 3038513 A1
- US 2018161266 A2
- WO 2021260486 A1
- WO 2020144440 A1
- WO 2018052709 A1
- WO 9600719 A
- WO 2018052709 A


### Littérature non-brevet citée dans la description


- **MICHEL** ; **IRENE ASH**. Thesaurus of Chemical Products. Chemical Publishing Co, Inc, 1986, vol. I, 211